(19) Europäisches Patentamt
European Patent Office
Office européen des brevets

(11) Veröffentlichungsnummer: **0 250 361 B1**

(12) **EUROPÄISCHE PATENTSCHRIFT**

(45) Veröffentlichungstag der Patentschrift: **08.12.93**

(51) Int. Cl.[5]: **C07D 295/18**, C07D 295/04, A61K 31/495

(21) Anmeldenummer: **87810331.6**

(22) Anmeldetag: **10.06.87**

Die Akte enthält technische Angaben, die nach dem Eingang der Anmeldung eingereicht wurden und die nicht in dieser Patentschrift enthalten sind.

(54) **Disubstituierte Piperazine.**

(30) Priorität: **16.06.86 CH 2420/86**

(43) Veröffentlichungstag der Anmeldung:
**23.12.87 Patentblatt 87/52**

(45) Bekanntmachung des Hinweises auf die Patenterteilung:
**08.12.93 Patentblatt 93/49**

(84) Benannte Vertragsstaaten:
**AT BE CH DE ES FR GB GR IT LI LU NL SE**

(56) Entgegenhaltungen:
**EP-A- 0 008 072** **EP-A- 0 042 366**
**EP-A- 0 098 713** **EP-A- 0 171 636**
**US-A- 3 000 892** **US-A- 3 917 617**

**CHEMICAL ABSTRACTS, Band 97, Nr. 1, 5. Juli 1985, Seite 6265, Zusammenfassung Nr. 6257p, Columbus, Ohio, US**

**CHEMICAL ABSTRACTS, Band 89, Nr. 5, 31. Juli 1978, Seite 21, Zusammenfassung Nr. 36473h, Columbus, Ohio, US**

**CHEMICAL ABSTRACTS, Band 83, Nr. 7, 18. August 1975, Seite 498, Zusammenfassung Nr. 58750z, Columbus, Ohio, US**

**CHEMICAL ABSTRACTS, Band 87, 1977, Zusammenfassung Nr. 33511s, Columbus, Ohio, US**

(73) Patentinhaber: **CIBA-GEIGY AG**
**Klybeckstrasse 141**
**CH-4002 Basel(CH)**

(72) Erfinder: **Ferrini, Pier Giorgio, Dr.**
**Im Rehwechsel 22**
**CH-4102 Binningen(CH)**
Erfinder: **Von Sprecher, Andreas, Dr.**
**Stallenmattstrasse 22**
**CH-4104 Oberwil(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Im US-Patent 3 917 617 werden bestimmte trisubstituierte Amide der Benzoesäure mit analgetischer, antidepressiver und antihypertensiver Wirkung offenbart. Zu diesen Amiden gehören auch zyklische Amide, z.B. Piperazide. In EP-A-42 366 werden 4-[4,4-(di-p-Fluorphenyl)butyl]-1-acyl-piperazine mit antiaggressiver, antipsychotischer, antidepressiver und analgetischer Wirkung offenbart. In EP-A-8 072 werden substituierte Anthranilsäureamide, insbesondere -piperazide, mit analgetischer, antiinflammatorischer und antiallergischer Wirkung offenbart. In Chem. Abstr. 87 (1977) 33511s wird unter anderem für 1-Phenylethyl-4-(3,4,5-trimethoxybenzoyl)-piperazin eine analgetische Wirkung offenbart. In Chem. Abstr. 97 (1982) 6257p wird unter anderem die Synthese von 1-Phenylethyl-4-(3,4,5-trimethoxybenzoyl)-piperazin offenbart.

Die Erfindung betrifft 1,4-disubsituierte Piperazine der Formel

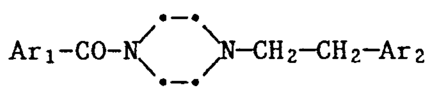

(I)

und ihre Salze, worin $Ar_1$ unsubstituiertes oder ein- oder zweifach durch $C_1$-$C_7$-Alkyl, $C_1$-$C_7$-Alkoxy, Cyano, Halogen, Trifluormethyl, Amino, $C_1$-$C_7$-Alkylamino, Di-$C_1$-$C_7$-alkylamino und/oder $C_1$-$C_7$-Alkanoylamino substituiertes Phenyl bedeutet, und worin $Ar_2$ unsubstituiertes oder ein- oder zweifach durch $C_1$-$C_7$-Alkyl, $C_1$-$C_7$-Alkoxy oder Halogen substituiertes Phenyl bedeutet, ihre Herstellung und Verwendung, pharmazeutische Präparate, enthaltend eine Verbindung der Formel I oder ein pharmazeutisch verwendbares Salz davon, und ihre Herstellung.

Die Verbindungen der Formel I können als, insbesondere pharmazeutisch verwendbare, Säureadditionssalze vorliegen. Diese werden beispielsweise mit starken anorganischen Säuren, wie Mineralsäuren, z.B. Schwefelsäure, Phosphorsäure oder Halogenwasserstoffsäuren, mit starken organischen Carbonsäuren, wie $C_1$-$C_4$-Alkancarbonsäuren, z.B. Essigsäure, wie gegebenenfalls ungesättigte Dicarbonsäuren, z.B. Oxal-, Malon-, Malein- oder Fumarsäure, oder wie Hydroxycarbonsäuren, z.B. Weinsäure oder Citronensäure, oder mit Sulfonsäuren, wie $C_1$-$C_4$-Alkan- oder gegebenenfalls substituierte Benzolsulfonsäure, z.B. Methan- oder p-Toluolsulfonsäure, gebildet. Entsprechende Säureadditionssalze können mit einem oder mehreren basischen Zentren gebildet werden, wobei z.B. entsprechende Mono- oder Dipiperaziniumsalze vorliegen.

Umfasst sind ferner für pharmazeutische Verwendungen nicht geeignete Salze, da diese beispielsweise für die Isolierung bzw. Reinigung freier erfindungsgemässer Verbindungen oder deren pharmazeutisch verwendbarer Salze verwendet werden können.

Die vor- und nachstehend verwendeten Allgemeindefinitionen haben, sofern nicht abweichend definiert, in erster Linie die folgenden Bedeutungen.

$C_1$-$C_7$-Alkyl ist z.B. Methyl, Ethyl, Propyl, Isopropyl, n-Butyl, Isobutyl, sek.-Butyl, tert.-Butyl und umfasst ferner entsprechende Pentyl-, Hexyl- und Heptylreste. Bevorzugt ist $C_1$-$C_4$-Alkyl.

$C_1$-$C_7$-Alkoxy ist insbesondere Methoxy, Ethoxy, Propyloxy, Isopropyloxy, Butyloxy, Isobutyloxy, sek.- und tert.-Butyloxy.

Halogen ist insbesondere Halogen mit Atomnummer bis und mit 35, wie Fluor, Chlor und Brom, ferner Iod.

$C_1$-$C_7$-Alkanoyl ist insbesondere Formyl, Acetyl, Propionyl, Butyryl oder Pivaloyl. Bevorzugt ist $C_2$-$C_5$-Alkanoyl.

Die neuen Verbindungen besitzen wertvolle pharmakologische Eigenschaften. So weisen sie vor allem analgetische Wirkungen auf, die sich z.B. anhand der Hemmung des durch Phenyl-p-Benzochinon ausgelösten Writhing-Syndroms der Maus ab einer Dosis von etwa 0,1 mg p.o. nachweisen lassen.

Die analgetische Aktivität zeigt sich ebenso im Essigsäure-Writhing-Test an der Ratte ab einer Dosis von etwa 0,1 mg/kg, wobei analog zur in Pain Res. and Therap. Vol. 1, 1976, page 517 (Raven Press N.A.) beschriebenen Methodik verfahren wurde.

Untersuchungen haben überraschenderweise ergeben, dass die erfindungsgemässen Verbindungen ein neuartiges Wirkungsprofil aufweisen. So wurde festgestellt, dass die Verbindungen der Formel I und ihre Salze keinen Einfluss auf die Arachidonsäure-Kaskade haben und z.B. entsprechend nicht den Prostaglandinsynthetasehemmern zuzuordnen sind. Auch lassen sich diese Verbindungen nicht dem Morphin-Typ zuordnen.

Dementsprechend können die Verbindungen der Formel I und ihre Salze z.B. als Analgetika zur Behandlung von Schmerzzuständen verwendet werden. Ein weiterer Gegenstand der Erfindung ist die Verwendung der erfindungsgemässen Verbindungen zur Herstellung von Arzneimitteln, insbesondere Analgetika, und zur therapeutischen und prophylaktischen Behandlung des menschlichen, ferner tierischen Körpers, insbesondere zur Behandlung von Schmerzzuständen. Dabei kann auch die gewerbsmässige Herrichtung der Wirksubstanzen eingeschlossen sein.

Die Erfindung betrifft insbesondere Verbindungen der Formel I und ihre Salze, worin $Ar_1$ unsubstituiertes oder ein- oder zweifach durch $C_1$-$C_4$-Alkyl, $C_1$-$C_4$-Alkoxy, Halogen, Trifluormethyl, Di-$C_1$-$C_4$-alkylamino und/oder $C_1$-$C_5$-Alkanoylamino substituiertes Phenyl bedeutet und $Ar_2$ unsubstituiertes oder ein- oder zweifach durch $C_1$-$C_4$-Alkoxy oder Halogen substituiertes Phenyl bedeutet.

Die Erfindung betrifft insbesondere Verbindungen der Formel I und ihre Salze, worin $Ar_1$ durch Halogen mit Atomnummer bis und mit 35, wie Fluor oder Chlor, insbesondere in 2- oder 4-Stellung, durch Trifluormethyl, insbesondere in 2- oder 3-Stellung, durch Di-$C_1$-$C_4$-alkylamino, wie Dimethylamino, insbesondere in 3-Stellung, oder durch $C_2$-$C_5$-Alkanoylamino, wie Acetylamino, insbesondere in 3-Stellung, jeweils monosubstituiertes Phenyl oder durch $C_1$-$C_4$-Alkyl, wie Methyl, insbesondere in 2- und 6-Stellung, oder durch $C_1$-$C_4$-Alkoxy, wie Methoxy, insbesondere in 3- und 4- oder 3- und 5-Stellung, jeweils disubstituiertes Phenyl bedeutet und $Ar_2$ durch Halogen mit Atomnummer bis und mit 35, wie Fluor oder Chlor, insbesondere in 4-Stellung, monosubstituiertes Phenyl bedeutet.

Die Erfindung betrifft insbesondere Verbindungen der Formel I und ihre Salze, worin $Ar_1$ unsubstituiertes oder ein- oder zweifach durch $C_1$-$C_4$-Alkyl, $C_1$-$C_4$-Alkoxy, Halogen und/oder Trifluormethyl substituiertes Phenyl bedeutet und $Ar_2$ durch Halogen monosubstituiertes Phenyl bedeutet.

Die Erfindung betrifft insbesondere Verbindungen der Formel I und ihre Salze, worin $Ar_1$ unsubstituiertes oder ein- oder zweifach durch $C_1$-$C_4$-Alkyl, $C_1$-$C_4$-Alkoxy, Halogen und/oder Trifluormethyl substituiertes Phenyl bedeutet und $Ar_2$ durch $C_1$-$C_4$-Alkoxy monosubstituiertes Phenyl bedeutet.

Die Erfindung betrifft in erster Linie Verbindungen der Formel I und ihre Salze, worin $Ar_1$ 3,5-Di-$C_1$-$C_4$-alkoxy-phenyl, insbesondere 3,5-Dimethoxyphenyl, bedeutet und $Ar_2$ 4-Halogenphenyl, insbesondere 4-Chlorphenyl, bedeutet.

Die Erfindung betrifft in erster Linie Verbindungen der Formel I und ihre Salze, worin $Ar_1$ 2- oder 4-Fluor-, 2- oder 4-Chlor-, 2- oder 3-Trifluormethyl-, 3- oder 4-Dimethylamino-, 3- oder 4-Acetylamino-, 3,4- oder 3,5-Dimethoxy- oder 2,6-Dimethylphenyl bedeutet und $Ar_2$ 4-Fluor- oder 4-Chlorphenyl bedeutet.

Die Erfindung betrifft in erster Linie Verbindungen der Formel I und ihre Salze, worin $Ar_1$ 2- oder 4-Fluor-, 3-Trifluormethyl-, 4-Chlor-, 3- oder 4-Dimethylamino- oder 3- oder 4-Acetylamino-phenyl bedeutet und $Ar_2$ 4-Fluor- oder 4-Chlorphenyl bedeutet.

Die Erfindung betrifft in erster Linie Verbindungen der Formel I und ihre Salze, worin $Ar_1$ durch Methyl, Methoxy, Fluor, Chlor oder Trifluormethyl monosubstituiertes oder durch Methoxy disubstituiertes Phenyl bedeutet und $Ar_2$ durch Fluor oder Chlor monosubstituiertes Phenyl bedeutet.

Die Erfindung betrifft in erster Linie Verbindungen der Formel I und ihre Salze, worin $Ar_1$ durch Methyl oder Fluor monosubstituiertes Phenyl bedeutet und $Ar_2$ durch Fluor oder Chlor monosubstituiertes Phenyl, insbesondere 4-Fluor- oder 4-Chlorphenyl, bedeutet.

Die Erfindung betrifft in erster Linie Verbindungen der Formel I und ihre Salze, worin $Ar_1$ 2- oder 4-Fluorphenyl, 2-Methylphenyl oder 3,5-Dimethoxyphenyl bedeutet und $Ar_2$ 4-Fluor- oder 4-Chlorphenyl bedeutet.

Die Erfindung betrifft insbesondere die in den Beispielen genannten neuen Verbindungen und Verfahren zu ihrer Herstellung.

Verfahren zur Herstellung der erfindungsgemässen Verbindungen sind ebenfalls Gegenstand der Erfindung. Die Herstellung von Verbindungen der Formel I und ihrer Salze erfolgt in an sich bekannter Weise und ist z.B. dadurch gekennzeichnet, dass man

a) eine Verbindung der Formel

$Ar_1$-$X_1$ (IIa)

oder ein Salz davon, worin $X_1$ Carboxy oder reaktionsfähiges funktionell abgewandeltes Carboxy bedeutet, mit einer Verbindung der Formel

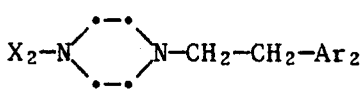 (IIb)

oder einem Salz davon umsetzt, worin $X_2$ Wasserstoff oder eine Aminoschutzgruppe bedeutet, oder

b) eine Verbindung der Formel

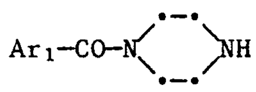

(IIIa)

oder ein Salz davon mit einer Verbindung der Formel

$X_3$-$CH_2$-$CH_2$-$Ar_2$ (IIIb)

oder einem Salz davon umsetzt, worin $X_3$ Hydroxy oder reaktionsfähiges verestertes Hydroxy bedeutet, oder

c) eine Verbindung der Formel

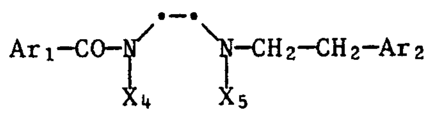

(IV)

oder ein Salz davon, worin einer der Reste $X_4$ und $X_5$ Wasserstoff ist und der andere für eine Gruppe der Formel -$CH_2$-$CH_2$-$X_3$ steht, und $X_3$ Hydroxy oder reaktionsfähiges verestertes Hydroxy bedeutet, intramolekular kondensiert oder

d) eine Verbindung der Formel

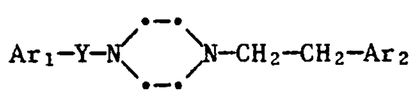

(V)

oder ein Salz davon, worin Y eine zu -CO- oxidierbare Gruppe bedeutet, oxidiert, oder,

e) zur Herstellung von Verbindungen der Formel I und ihrer Salze, worin $Ar_1$ durch $C_1$-$C_7$-Alkoxy mono- oder disubstituiertes Phenyl bedeutet, eine Verbindung der Formel

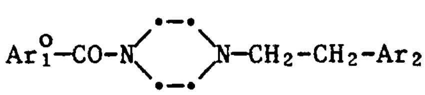

(VI)

oder ein Salz davon, worin $Ar_1^o$ durch Hydroxy mono- oder disubstituiertes Phenyl bedeutet, alkyliert und gewünschtenfalls eine verfahrensgemäss oder auf andere Weise erhältliche Verbindung in eine andere Verbindung der Formel I überführt, ein verfahrensgemäss erhältliches Isomerengemisch in die Komponenten auftrennt, eine verfahrensgemäss erhältliche freie Verbindung der Formel I in ein Salz überführt und/oder ein verfahrensmäss erhältliches Salz in die freie Verbindung der Formel I oder in ein anderes Salz überführt.

Die vor- und nachstehend in den Varianten beschriebenen Umsetzungen werden in an sich bekannter Weise durchgeführt, z.B. in Ab- oder üblicher Weise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -78° bis zur Siedetemperatur des Reaktionsmediums, vorzugsweise von etwa -10° bis etwa 150°C, und, falls erforderlich, in einem geschlossenen Gefäss, unter Druck, in einer Inertgasatmosphäre und/oder unter wasserfreien Bedingungen arbeitet.

Das vor- und nachstehend aufgeführte Ausgangsmaterial der Formeln IIa und IIb, IIIa und IIIb, IV, V sowie VI, das für die Herstellung der Verbindungen der Formel I und deren Salze entwickelt wurde, ist zum Teil bekannt oder kann ebenfalls nach an sich bekannten Methoden, z.B. analog den vorstehend beschriebenen Verfahrensvarianten, hergestellt werden.

In den Ausgangsmaterialien kann das basische Zentrum z.B. in Form von Säureadditionssalzen, beispielsweise mit den vorstehend im Zusammenhang mit Salzen von Verbindungen der Formel I aufgeführten Säuren, vorliegen, während Ausgangsverbindungen mit sauren Gruppen, Salze mit Basen bilden können. Geeignete Salze mit Basen sind beispielsweise entsprechende Alkalimetall- oder Erdalkalimetallsalze, z.B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclische Amine, wie Mono-, Di- bzw. Trihydroxy-$C_1$-$C_7$-alkylamine, Hydroxy-$C_1$-$C_7$-alkyl-$C_1$-$C_7$-alkyl-amine oder wie Polyhydroxy-$C_4$-$C_7$-alkylamine. Cyclische Amine sind z.B. Morpholin, Thiomorpholin, Piperidin oder Pyrrolidin. Als Mono-$C_1$-$C_7$-alkylamine kommen beispielsweise Ethyl- oder tert.-Butylamin, als Di-$C_1$-$C_7$-alkylamine beispielsweise Diethyl- oder Diisopropylamin, als Tri-$C_1$-$C_7$-alkylamine beispielsweise Trimethyl- oder Triethylamin in Betracht. Entsprechende Hydroxy-$C_1$-$C_7$-alkylamine sind z.B. Mono-, Di- bzw. Triethanolamine, und Hydroxy-$C_1$-$C_7$-alkyl-$C_1$-$C_7$-alkylamine sind z.B. N,N-Dimethylamino- oder N,N-Diethylaminoethanol, ferner Glucosamin als Polyhydroxy-$C_6$-alkylamin.

Reaktionsfähiges funktionell abgewandeltes Carboxy $X_1$ bedeutet beispielsweise verestertes, in erster Linie reaktionsfähiges verestertes, Carboxy, anhydridisiertes Carboxy oder amidiertes Carboxy.

Verestertes Carboxy ist beispielsweise gegebenenfalls substituiertes $C_1$-$C_7$-Alkoxycarbonyl, wie Ethoxycarbonyl, vorzugsweise jedoch reaktionsfähiges verestertes Carboxy, beispielsweise gegebenenfalls, z.B. durch $C_1$-$C_7$-Alkoxy oder gegebenenfalls substituiertes Carbamoyl, zusätzlich aktiviertes Vinyloxycarbonyl, wie 1-$C_1$-$C_7$-Alkoxy-, z.B. 1-Ethoxyvinyloxycarbonyl, oder 2-(N-$C_1$-$C_7$-Alkyl-carbamoyl)-, z.B. 2-(N-Ethylcarbamoyl)-vinyloxycarbonyl, sowie gegebenenfalls, z.B. durch Nitro, Halogen, $C_1$-$C_7$-Alkansulfonyl oder Phenylazo, substituiertes Phenoxy- bzw. Thiophenoxycarbonyl, wie 4-Nitro-, 2,4,5-Trichlor-, Pentachlor-, 4-Methansulfonyl-, 4-Phenylazo-phenoxycarbonyl, Thiophenoxy- oder 4-Nitrothiophenoxycarbonyl, und ebenso aktiviertes, z.B. durch Cyano oder ferner gegebenenfalls vererstertes Carboxy substituiertes, Methoxycarbonyl, insbesondere Cyanomethoxycarbonyl. Reaktionsfähiges verestertes Carboxy kann ebenso 1,1- oder 1,3-disubstituiertes 2-Isoureidocarbonyl, wie 1,1-Diniederalkyl-, 1,1-Diaryl- oder 1,1-Diaryl-$C_1$-$C_7$-alkyl-2-isoureidocarbonyl, z.B. 1,1-Diethyl-, 1,1-Diphenyl- oder 1,1-Dibenzyl-2-isoureidocarbonyl, oder 1,3-Dicycloalkyl-, z.B. 1,3-Dicyclohexyl-2-isoureido-carbonyl, oder N-$C_2$-$C_7$-Alkylenaminooxycarbonyl, wie N-Piperidinyl-oxycarbonyl, sowie N-Imido-oxycarbonyl, z.B. N-Succinimido-oxy- oder N-Phthalimido-oxycarbonyl, sein.

Unter anhydridisiertem Carboxy ist beispielsweise gegebenenfalls verzweigtes $C_1$-$C_7$-Alkoxycarbonyloxycarbonyl, wie Ethoxy- oder Isobutyloxycarbonyloxycarbonyl, Halogencarbonyl, wie Chlorcarbonyl, Azidocarbonyl, Halogenphosphoryloxycarbonyl, wie Dichlorphosphoryloxycarbonyl, oder gegebenenfalls, z.B. durch Halogen oder Aryl, substituiertes $C_1$-$C_7$-Alkanoyloxycarbonyl, wie Pivaloyloxy-, Trifluoracetyloxy- oder Phenylacetoxycarbonyl, zu verstehen. Anhydridisiertes Carboxy kann ferner symmetrisch anhydridisiertes Carboxy der Formel $Ar_1$-CO-O-CO- sein.

Reaktionsfähiges amidiertes Carboxy ist beispielsweise gegebenenfalls, z.B. durch $C_1$-$C_7$-Alkyl, substituiertes 1-Imidazolyl- oder 1-Pyrazolylcarbonyl, wie 3,5-Dimethyl-pyrazolylcarbonyl.

Eine Aminoschutzgruppe $X_2$ ist beispielsweise Acyl, wie $C_1$-$C_7$-Alkanoyl, z.B. Formyl oder Acetyl, Halogencarbonyl, wie Chlorcarbonyl, ferner gegebenenfalls substituiertes (Hetero)Arylsulfonyl, wie 2-Pyridyl- oder 2-Nitrophenylsulfenyl.

Im Rahmen der vor- und nachstehenden Verfahrensbeschreibung bedeutet reaktionsfähiges verestertes Hydroxy, z.B. $X_3$, sofern nicht abweichend definiert, insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes $C_1$-$C_7$-Alkansulfonyloxy, z.B. Methan-oder Trifluormethansulfonyloxy, $C_3$-$C_7$-Cycloalkansulfonyloxy, z.B. Cyclohexansulfonyloxy, oder gegebenenfalls, z.B. durch $C_1$-$C_7$-Alkyl oder Halogen, substituiertes Benzolsulfonyloxy, z.B. p-Bromphenyl- oder p-Toluolsulfonyloxy.

Werden bei den vor- und nachstehend beschriebenen Umsetzungen beispielsweise Basen verwendet, so kommen, sofern nicht abweichend aufgeführt, beispielsweise Alkalimetallhydroxide, -hydride, -amide, -alkanolate, -carbonate, -triphenylmethylide, -di-$C_1$-$C_7$-alkylamide, -amino-$C_1$-$C_7$-alkylamide oder -$C_1$-$C_7$-alkylsilylamide, Naphthalinamine, $C_1$-$C_7$-Alkylamine, basische Heterocyclen, Ammoniumhydroxide sowie carbocyclische Amine in Frage. Beispielhaft seien Lithium-hydroxid, Natrium-hydroxid, -hydrid, -amid, -ethylat, Kalium-tertbutylat, -carbonat, Lithiumtriphenylmethylid, -diisopropylamid, Kalium-3-(aminopropyl)-amid, -bis-(trimethylsilyl)-amid, Dimethylaminonaphthalin, Di- oder Triethylamin, Pyridin, Benzyl-trimethylammoniumhydroxid, 1,5-Diaza-bicyclo[4.3.0]non-5-en (DBN) sowie 1,8-Diaza-bicyclo[5.4.0]undec-7-en (DBU) genannt.

Variante a):

Die verfahrensgemässe N-Acylierung wird in an sich bekannter Weise durchgeführt, erforderlichenfalls in Gegenwart eines, insbesondere basischen, Kondensationsmittels. Als Basen kommen beispielsweise Vertreter der vorstehend aufgeführten Basen in Frage. Häufig ist auch die Basizität der Verbindung der Formel IIb ausreichend.

Falls $X_1$ Carboxy bedeutet, werden z.B. primär die entsprechenden Ammoniumsalze gebildet, die durch Erwärmen oder Behandeln mit geeigneten Dehydratisierungsmitteln (als Kondensationsmittel), wie Carbodiimiden, z.B. N,N'-Diniederalkyl- oder N,N'-Dicycloalkylcarbodiimid, wie N,N'-Diethyl-, N,N'-Diisopropyl- oder N,N'-Dicyclohexyl-carbodiimid, vorteilhaft unter Zusatz von N-Hydroxysuccinimid oder gegebenenfalls, z.B. durch Halogen, Niederalkoxy oder Niederalkyl, substituiertem 1-Hydroxy-benzotriazol oder N-Hydroxy-5-norbornen-2,3-dicarboxamid, sowie N,N-Carbonyldiimidazol, dehydratisiert werden können. Mit Carbodiimiden können intermediär z.B. auch die entsprechenden 1-Isoureidocarbonyl-Verbindungen gebildet werden. Als wasserbindende Kondensationsmittel können weiterhin N-Ethoxycarbonyl-2-ethoxy-1,2-dihydrochinolin, Phosphorylcyanamide bzw. -azide, wie Diethylphosphorylcyanamid oder Diphenylphosphorylazid, Triphenylphosphin-disulfid oder 1-Niederalkyl-2-halogeno-piperidinium-halogenide, wie 1-Methyl-2-chlorpyridinium-iodid, eingesetzt werden.

Weisen Verbindungen der Formel IIa eine durch Amino in Position 2 substituiertes Phenyl auf und ist $X_1$ Carboxy, kann eine derartige Verbindung der Formel IIa als ein entsprechendes Isatosäureanhydrid vorliegen.

Die in dieser Verfahrensvariante verwendeten Ausgangsstoffe sind z.T. bekannt bzw. können nach an sich bekannten Verfahren hergestellt werden.

Zur Herstellung von Verbindungen der Formel (IIa), worin $X_1$ gegebenenfalls substituiertes $C_1$-$C_7$-Alkoxycarbonyl bedeutet, kann man üblicherweise von der freien Säure ($X_1$ = Carboxy) oder einem Säureanhydrid ($X_1$ bedeutet z.B. Halogencarbonyl) ausgehen und setzt beispielsweise mit dem entsprechenden, erforderlichenfalls in reaktionsfähiger Form vorliegenden, Alkohol, z.B. einem $C_1$-$C_7$-Alkylhalogenid, um. Die Herstellung von Verbindungen der Formel (IIa), worin $X_1$ gegebenenfalls zusätzlich aktiviertes Vinyloxycarbonyl bedeutet, kann z.B. durch Umesterung eines $C_1$-$C_7$-Alkylesters mit Vinylacetat (Methode des aktivierten Vinylesters), durch Umsetzung der freien Säure von Verbindungen der Formel (IIa) mit Niederalkoxyacetylen (z.B. Ethoxyacetylen-Methode) oder, analog der Woodward-Methode, mit einem 1,2-Oxazoliumsalz erfolgen. Gegebenenfalls substituiertes Phenoxy- bzw. Thiophenoxycarbonyl aufweisende Verbindungen der Formel (IIa) können beispielsweise ausgehend von der freien Säure nach der Carbodiimid-Methode durch Umsetzen mit dem entsprechenden (Thio-)Phenol durchgeführt werden. Ebenfalls ausgehend von der freien Säure der Formel (IIa) können Verbindungen der Formel (IIa), worin $X_1$ aktiviertes Methoxycarbonyl bzw. 1,1- oder 1,3-disubstituiertes 2-Isoureidocarbonyl darstellt, z.B. durch Umsetzung mit einem Halogen-, wie Chloracetonitril (Cyanmethylester-Methode) bzw. mit einem Carbodiimid oder Cyanamid (Carbodiimid- oder Cyamid-Methode) erhalten werden. Die Herstellung von N-$C_2$-$C_7$-Alkylenamino-oxycarbonyl-bzw. N-Imido-oxycarbonyl-Verbindungen der Formel (IIa) kann beispielsweise bei Verwendung der freien Säure der Formel (IIa) aus entsprechenden N-Hydroxyverbindungen mit Hilfe von Carbodiimiden nach der Methode der aktivierten N-Hydroxyester erfolgen. Zur Herstellung von Verbindungen der Formel (IIa), worin $X_1$ gegebenenfalls verzweigtes $C_1$-$C_7$-Alkoxycarbonyloxycarbonyl, Halogenphosphoryloxycarbonyl bzw. gegebenenfalls substituiertes $C_1$-$C_7$-Alkanoyloxycarbonyl bedeutet, kann beispielsweise von freier Säure der Formel (IIa) ausgegangen werden, die z.B. mit einem entsprechenden Halogenid, wie gegebenenfalls substituiertes $C_1$-$C_7$-Alkylkohlensäurehalogenid (Methode der gemischten O-Kohlensäureanhydride), Phosphoroxyhalogenid (z.B. Phosphoroxychlorid-Methode) oder gegebenenfalls substituiertes $C_1$-$C_7$-Alkanoylhalogenid (Methode der gemischten Carbonsäurehalogenide) behandelt werden kann. Azidocarbonylverbindungen der Formel (IIa) sind beispielsweise durch Behandeln entsprechender Hydrazide mit salpetriger Säure zugänglich (Azid-Methode). Zur Herstellung von Verbindungen der Formel (IIa), worin $X_1$ gegebenenfalls substituiertes 1-Imidazoyly-carbonyl bzw. 1-Pyrazolyl-carbonyl bedeutet, setzt man die freie Säure der Formel (IIa) z.B. mit Di-(1-imidazolyl)-carbonyl (Imidazolid-Methode) bzw. das betreffende Hydrazid z.B. mit einem entsprechenden 1,3-Diketon (Pyrazolid-Methode) um.

Variante b):

$X_3$ bedeutet insbesondere reaktionsfähiges verestertes Hydroxy, bevorzugt Halogen, wie Chlor.

Die verfahrensgemässe N-Alkylierung wird in an sich bekannter Weise durchgeführt, erforderlichenfalls in Gegenwart einer, z.B. der vorstehend aufgeführten, Basen.

Die in dieser Verfahrensvariante verwendeten Ausgangsstoffe sind z.T. bekannt oder können in an sich bekannter Weise hergestellt werden.

So kann beispielsweise das Ausgangsmaterial der Formel IIIa hergestellt werden, indem man eine Verbindung der Formel $Ar_1$-$X_1$ (IIa) oder ein Salz davon, worin $X_1$ Carboxy oder reaktionsfähiges funktionell abgewandeltes Carboxy bedeutet, mit einer Verbindung der Formel

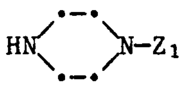 (IIIc)

oder einem Salz davon, worin $Z_1$ Wasserstoff oder eine Aminoschutzgruppe, wie Benzyl, bedeutet, in der in Variante a) beschriebenen Weise umsetzt und gegebenenfalls die Aminoschutzgruppe abspaltet, z.B. Benzyl durch übliche Hydrogenolyse.

Variante c):

Die verfahrensgemässe intramolekulare N-Alkylierung wird in an sich bekannter Weise, erforderlichenfalls in Gegenwart eines, insbesondere basischen, Kondensationsmittels durchgeführt. Als Basen werden z.B. vorstehend aufgeführte Vertreter verwendet.

$X_3$ bedeutet insbesondere reaktionsfähiges verestertes Hydroxy, bevorzugt Halogen, wie Chlor.

Das Ausgangsmaterial kann in an sich bekannter Weise hergestellt werden. Beispielsweise geht man von einer Verbindung der Formel $Ar_1$-$X_1$ (IIa) oder einem Salz davon aus, worin $X_1$ Carboxy oder reaktionsfähiges funktionell abgewandeltes Carboxy bedeutet, und setzt diese zunächst mit einer Verbindung der Formel $Ar_2$-$CH_2$-$CH_2$-NH-$CH_2$-$CH_2$-$NH_2$ (IVa) in Analogie zu Variante a) um. Im nächsten Reaktionsschritt wird die erhaltene Verbindung mit einer Verbindung der Formel

$X_3$-$CH_2$-$CH_2$-$X_3$ (IVb)

unter N-alkylierenden Bedingungen gemäss Variante b) umgesetzt.

Variante d):

Eine zu -CO- oxidierbare Gruppe Y steht in erster Linie für -$CH_2$-.

Die Oxidation entsprechender Verbindungen der Formel V erfolgt mit Hilfe eines geeigneten Oxidationsmittels, wobei bevorzugt gegebenenfalls, z.B. durch einen Phenylrest, substituierte Tetra-$C_1$-$C_4$-alkylammoniumpermanganate, in erster Linie Benzyl-triethyl-ammoniumpermanganat, verwendet wird.

Das Ausgangsmaterial der Formel V wird in an sich bekannter Weise hergestellt. Beispielsweise geht man von einer Verbindung der Formel IIb aus, worin $X_2$ Wasserstoff bedeutet, und setzt diese unter den in Variante b) beschriebenen N-alkylierenden Bedingungen mit einer Verbindung der Formel $Ar_1$-$CH_2$-$X_6$ (Va) um, wobei $X_6$ Hydroxy oder in erster Linie reaktionsfähiges verestertes Hydroxy, insbesondere Halogen, wie Chlor oder Brom, bedeutet.

Variante e):

Die Alkylierung kann beispielsweise mit Hilfe eines entsprechenden an sich bekannten Alkylierungsmittels erfolgen. Als derartige Mittel kommen z.B. $C_1$-$C_7$-Alkanole und insbesondere reaktionsfähige veresterte Derivate davon in Frage. Vorzugsweise kommen $C_1$-$C_7$-Alkylhalogenide, wie Methyliodid, ebenso Di-$C_1$-$C_7$-alkylsulfate, wie Dimethylsulfat, Diazo-$C_1$-$C_4$-alkane, wie Diazomethan, $C_1$-$C_7$-Alkylsulfonate, wie entsprechende gegebenenfalls durch Halogen oder $C_1$-$C_7$-Alkyl substituierte Benzolsulfonate, z.B. entsprechende p-Bromphenyl- oder p-Toluolsulfonate, Tri-$C_1$-$C_7$-alkylsulfonium-, Tri-$C_1$-$C_7$-alkylselenium-, Tri-$C_1$-$C_7$-alkyloxosulfonium- oder Tri-$C_1$-$C_7$-alkylanilinium-hydroxide, wie Trimethylsulfonium-, Trimethylselenium-, Trimethyloxosulfonium- oder Trimethylaniliniumhydroxid, zur Anwendung.

Bei der Verwendung von reaktionsfähigen veresterten Derivaten von $C_1$-$C_7$-Alkanolen, beispielsweise eines Di-$C_1$-$C_7$-alkylsulfats, erfolgt die Alkylierung insbesondere in Gegenwart einer der vorstehend genannten Basen, vorzugsweise von Kaliumhydroxid oder -carbonat, während die Umsetzung mit einem Diazo-$C_1$-$C_4$-alkan erforderlichenfalls in Gegenwart einer Lewissäure durchgeführt wird. Lewissäuren sind beispielsweise Halogenide von Bor, Aluminium, Zinn (II), Antimon (III), Arsen (III), Silber (I), Zink (II) und Eisen (III).

Die Alkylierung mit einem $C_1$-$C_7$-Alkanol, wie Methanol, wird beispielsweise in Gegenwart einer der vorstehend aufgeführten Säuren durchgeführt.

Zur Herstellung des Ausgangsmaterials der Formel VI geht man beispielsweise von einer Verbindung der Formel $Ar_1^o$ -$X_1$ (VIa) aus, schützt die Hydroxygruppe(n) durch Einführung einer Hydroxyschutzgruppe, z.B. durch Acylierung, wie Acetylierung mit Acetonhydrid, und setzt die so erhältliche Verbindung in Analogie zu Variante a) mit gegebenenfalls einfach geschütztem Piperazin um.

Eine verfahrensgemässe oder anderweitig erhältliche erfindungsgemässe Verbindung kann in an sich bekannter Weise in eine andere erfindungsgemässe Verbindung übergeführt werden.

In erfindungsgemässen Verbindungen, in welchen der Rest $Ar_1$ bzw. $Ar_2$ eine freie Aminogruppe enthält, kann die jeweilige Aminogruppe in der vorstehend unter Variante a) oder b) angegebenen Weise mono-oder disubstituiert, d.h. N-acyliert bzw. N-alkyliert, werden. Ebenso können primäre oder sekundäre Aminogruppen analog der Leuckart-Wallach- (bzw. Eschweiler-Clarke)-Reaktion aus Carbonylverbindungen, z.B. unter Verwendung von Ameisensäure als Reduktionsmittel, reduktiv alkyliert werden.

Verbindungen der Formel I, worin $Ar_1$ und/oder $Ar_2$ durch Amino substituiertes Phenyl bedeutet, können in an sich bekannter Weise in solche Verbindungen der Formel I übergeführt werden, worin $Ar_1$ und/oder $Ar_2$ durch $C_1$-$C_7$-Alkoxy, Cyano oder Halogen, insbesondere Chlor oder Fluor, ferner Brom oder Jod, substituiertes Phenyl bedeutet. Hierzu wird beispielsweise die Aminogruppe z.B. durch Behandeln mit einem Nitrit, wie Alkalimetallnitrit, z.B. Natriumnitrit, oder mit Nitroniederalkanen in Gegenwart von Protonsäuren zur Diazoniumgruppe -$N_2^{\oplus}A^{\ominus}$ diazotiert, wobei $A^{\ominus}$ für ein Anion, wie Chlorid, steht. Die Diazoniumgruppe kann im nächsten Reaktionsschritt z.B. durch Umsetzung mit Cyaniden, z.B. analog der Sandmeyer-Reaktion mit Kupfer(I)cyanid oder Alkalimetall-tetracyano-cuprat (I) oder in Anlehnung an die Gattermann-Reaktion mit Alkalimetallcyaniden in Gegenwart von metallischem Kupfer, durch Cyano ersetzt werden. Die Substitution der Gruppe -$N_2^{\oplus}A^{\ominus}$ durch Halogen kann beispielsweise durch Behandeln mit Halogeniden, wie Kupfer(I)halogeniden z.B. gemäss der Sandmeyer-Reaktion oder mit Alkalimetallhalogeniden in Gegenwart von metallischem Kupfer analog der Gatterman-Reaktion erfolgen. Fluor kann ausserdem beispielsweise durch Umsetzung mit einem Tetrafluoroboranat gemäss der Schiemann-Reaktion eingeführt werden. Behandelt man entsprechende Diazoniumverbindungen mit einem $C_1$-$C_7$-Alkanol, kann $C_1$-$C_7$-Alkoxy eingeführt werden. In einer vorteilhaften Modifikation dieser Umwandlungsreaktionen werden entsprechende Diazoniumverbindungen in situ gebildet und reagieren unter den jeweiligen Reaktionsbedingungen ohne Isolierung zu den entsprechenden Verbindungen der Formel I weiter.

Salze der Formel (I) können in an sich bekannter Weise hergestellt werden. So erhält man beispielsweise Säureadditionssalze von freien Verbindungen der Formel (I) durch Behandeln mit einer Säure oder einem geeigneten Ionenaustauscherreagenz. Salze können in üblicher Weise in die freien Verbindungen überführt werden, Säureadditionssalze z.B. durch Behandeln mit einem geeigneten basischen Mittel.

Je nach Verfahrensweise bzw. Reakstionsbedingungen können die erfindungsgemässen Verbindungen mit salzbildenden, insbesondere basischen Eigenschaften, in freier Form oder in Form von Salzen erhalten werden.

Infolge der engen Beziehung zwischen der neuen Verbindung in freier Form und in Form ihrer Salze sind im vorausgegangenen und nachfolgend unter der freien Verbindung oder ihren Salzen sinn- und zweckgemäss gegebenenfalls auch die entsprechenden Salze bzw. die freie Verbindung zu verstehen.

Die neuen freien Verbindungen einschliesslich ihrer Salze können auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Racematgemische können auf Grund der physikalisch-chemischen Unterschiede der Bestandteile in bekannter Weise in die reinen Isomeren oder Racemate getrennt aufgetrennt werden, beispielsweise durch fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein- oder Aepfelsäure, odeer Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden

kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, beispielsweise der Formeln IIa, IIb, IIIa, IIIb, IV, V und VI, die speziell für die Herstellung der erfindungsgemässen Verbindungen entwickelt wurden, ihre Verwendung und Verfahren zu ihrer Herstellung bilden ebenfalls einen Gegenstand der Erfindung, wobei die Variablen $Ar_1$ und $Ar_2$ die für die jeweils bevorzugten Verbindungsgruppen der Formel I angegebenen Bedeutungen haben.

Ein weiterer Gegenstand der Erfindung sind Ausgangsverbindungen der Formel

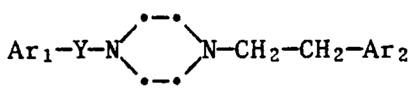

(V)

und ihre Salze, worin Y für $-CH_2-$ steht und $Ar_1$ und $Ar_2$ die für $Ar_1$ und $Ar_2$ der Formel I angegebenen Bedeutungen haben, ihre Herstellung und Verwendung, z.B. als Ausgangsmaterialien für die Herstellung von Verbindungen der Formel I, pharmazeutische Präparate, enthaltend eine derartige Verbindung der Formel V oder ein pharmazeutisch verwendbares Salz davon. Entsprechende Verbindungen der Formel V weisen ebenfalls ausgeprägte analgetische Eigenschaften auf. In bevorzugten Verbindungen der Formel V und ihrer Salze haben die Variabeln $Ar_1$ und $Ar_2$ die für die jeweils bevorzugten Verbindungsgruppen der Formel I angegebenen Bedeutungen.

Die Erfindung betrifft ebenfalls die Verwendung der Verbindungen der Formel (I) oder von pharmazeutisch verwendbaren Salzen von solchen Verbindungen mit salzbildenden Eigenschaften, insbesondere als pharmakologische, in erster Linie analgetisch wirksame, Wirksubstanzen. Dabei kann man sie, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Analgetika, z.B. zur Behandlung von Schmerzzuständen, wie Migräne, verwenden.

Die Erfindung betrifft gleichfalls pharmazeutische Präparate, die die erfindungsgemässen Verbindungen oder pharmazeutisch verwendbare Salze derselben als Wirkstoffe enthalten, sowie Verfahren zu ihrer Herstellung.

Bei den erfindungsgemässen pharmazeutischen Präparaten, welche die erfindungsgemässe Verbindung oder pharmazeutisch verwendbare Salze davon enthalten, handelt es sich um solche zur enteralen, wie oralen ferner rektalen, und parenteralen sowie topischen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffs hängt von dem Alter und dem individuellen Zustand sowie von der Applikationsweise ab.

Die neuen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferne Bindemittel, wie Stärkekleister, unter Verwendung von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetzes Polyvinylpyrrolidon, Agar, Alginsäure oder ein Salz davon, wie Natriumalginat, Hilfsmittel sind in erster Linie Fliess-, Regulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen

Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaftresistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositorienmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffs hängt von der Warmblüter-Spezis, dem Alter und dem individuellen Zustand sowie der Applikationsweise ab. Im Normalfall ist für einen etwa 75 kg schweren Warmblüter bei oraler Applikation eine ungefähre Tagesdosis von etwa 100 mg bis etwa 1000 mg. vorteilhaft in mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden und Druck in Pa angegeben.

Beispiel 1: Eine bei Raumtemperatur gerührte Lösung von 5 g 1-[2-(4-Chlorphenyl)-ethyl]-piperazin in 75 ml Methylenchlorid wird tropfenweise während 10 Min. mit einer Lösung von 4,65 g 4-Chlorbenzoylchlorid in 50 ml Methylenchlorid versetzt. Das Reaktionsgemisch wird 5 Min. zum Rückfluss erhitzt, 1 Stunde bei Raumtemperatur stehen gelassen und eingedampft. Der Rückstand wird in heissem Aceton aufgeschlämmt und nach der Zugabe von Hexan filtriert. Man erhält 1-[2-)3-Chlorphenyl)-ethyl]-4-(4-chlorbenzoyl)-piperazin, das über das Hydrochlorid vom Smp. 254-254,5° (Zers.) gereinigt und charakterisiert werden kann.

Beispiel 2: In analoger Weise, wie in Beispiel 1 beschrieben, erhält man aus 3-Chlorbenzoylchlorid das 1-[2-(4-Chlorphenyl)-ethyl]-4-(3-chlorbenzoyl)-piperazin-hydrochlorid vom Smp. 221-223° (Zers.), 2-Chlorbenzoylchlorid das 1-[2-((4-Chlorphenyl)-ethyl]-4-(2-chlorbenzoyl)-piperazin-hydrochlorid vom Smp. 230-233° (Zers.), 2,6-Dichlorbenzoylchlorid das 1-[2-(4-Chlorphenyl)-ethyl]-4-(2,6-dichlorbenzoyl)-piperazin-hydrochlorid vom Smp. ab 250° (Zers.), 2-Fluorbenzoylchlorid das 1-[2-(4-Chlorphenyl)-ethyl]-4-(2-fluorbenzoyl)-piperazin-hydrochlorid vom Smp. 215-218°, 2,4-Dichlorbenzoylchlorid das 1-[2-(4-Chlorphenyl)-ethyl]-4-(2,4-dichlorbenzoyl)-piperazinhydrochlorid vom Smp. ab 240° (Zers.), 4-Fluorbenzoylchlorid das 1-[2-(4-Chlorphenyl)-ethyl]-4-(4-fluorbenzoyl)-piperazin-hydrochlorid vom Smp. ab 222° (Zers.), 3,4-Dichlorbenzoylchlorid das 1-[2-(4-Chlorphenyl)-ethyl]-4-(3,4-dichlorbenzoyl)-piperazin-hydrochlorid vom Smp. ab 240° (Zers.) und 3-Fluorbenzoylchlorid das 1-[2-(4-Chlorphenyl)-ethyl]-4-(3-fluorbenzoyl)-piperazin-hydrochlorid vom Smp. 195-198° (Zers.).

Beispiel 3: 4,7 g Isatosäureanhydrid werden in 60 ml abs. Dioxan auf 80-90° erhitzt. Dazu gibt man eine Lösung von 6,3 g 1-[2-(4-Chlorphenyl)-ethyl]-piperazin in 70 ml abs. Dioxan. Man kocht 1 Stunde unter Rückfluss, dampft im Vakuum zur Trockene ein und chromatographiert das viskose Oel an Kieselgel. Das Methylenchlorid-Aceton (1:1)-Eluat wird eingedampft, in Methanol gelöst und das erhaltene 1-[2-(4-Chlorphenyl)-ethyl]-4-(2-aminobenzoyl)-piperazin mit methanolischer Salzsäure in das Hydrochlorid überführt. Man erhält 1-[2-(4-Chlorphenyl)-ethyl]-4-(2-aminobenzoyl)-piperazinhydrochlorid vom Smp. 238-240°.

Beispiel 4: In analoger Weise, wie in Beispiel 3 beschrieben, erhält man aus 8,7 g Isatosäureanhydrid und 10,1 g 1-(2-Phenylethyl)-piperazin direkt, ohne Chromatographie, das 1-(2-Phenylethyl)-4-(2-amino-benzoyl)-piperazin vom Smp. 124-126°.

Beispiel 5: 11,23 g 1-[2-(4-Chlorphenyl)-ethyl]-piperazin werden in 100 ml Methylenchlorid gelöst, mit 9 ml Triethylamin und dann tropfenweise unter Aussenkühlung mit 11,08 g 3-Methoxybenzoylchlorid in 100 ml Methylenchlorid versetzt. Man rührt die Reaktionsmischung über Nacht bei Raumtemperatur weiter und schüttelt sie dann einmal mit 50 ml 2N-Natronlauge und zweimal mit je 100 ml Wasser aus. Nach dem Trocknen über Natriumsulfat wird die Mischung im Vakuum zum Trocknen eingedampft. Das ölige 1-[2-(4-Chlorphenyl)-ethyl]-4-(3-methoxybenzyol)-piperazin wird in Isopropanol gelöst und mit alkoholischer Salzsäure versetzt. Nach Zugabe von Ether wird das Kristallisat filtriert. Man erhält 1-[2-(4-Chlorphenyl)-ethyl]-4-(3-methoxy-benzoyl)-piperazin-hydrochlorid vom Smp. 230-232°.

Beispiel 6: In analoger Weise, wie in Beispiel 5 beschrieben, wird aus 11,23 g 1-[2-(4-Chlorphenyl)-ethyl]-piperazin und 13 g 3,4-Dimethoxybenzoylchlorid das 1-[2-(4-Chlorphenyl)-ethyl]-4-(3,4-dimethoxybenzoyl)-piperazin vom Smp. 123-125° erhalten.

Beispiel 7: 22,4 g 1-[2-(4-Chlorphenyl)-ethyl]-piperazin und 12,1 g Triethylamin werden in 200 ml Dichlormethan gelöst und unter Rühren bei 25-30° tropfenweise mit einer Lösung von 20,4 g 4-Methoxybenzoylchlorid in 30 ml Dichlormethan versetzt. Man lässt über Nacht bei Raumtemperatur nachrühren, wäscht nacheinander mit 150 ml 2n-Natronlauge und Wasser, trocknet über Natriumsulfat und dampft zur Trockne ein. Der Eindampfrückstand wird aus Ethanol kristallisiert. Man erhält das 1-[2-(4-Chlorphenyl)-ethyl]-4-(4-methoxybenzoyl)-piperazin vom Smp. 105-106°.

Beispiel 8: In analoger Weise wie in Beispiel 7 beschrieben kann man ausgehend von 22,4 g 1-[2-(4-Chlorphenyl)-ethyl]-piperazin und 18,6 g 4-Methylbenzoylchlorid das 1-[2-(4-Chlorphenyl)-ethyl]-4-(4-methylbenzoyl)-piperazin vom Smp. 105-106° herstellen.

Beispiel 9: In analoger Weise in Beispiel 7 beschrieben kann man ausgehend von 22,4 g 1-[2-(4-Chlorphenyl)-ethyl]-piperazin und 12,7 g 2-Methoxybenzoylchlorid das 1-[2-(4-Chlorphenyl)-ethyl]-4-(2-methoxybenzoyl)-piperazin vom Smp. 114-115° herstellen.

Beispiel 10: In analoger Weise wie in Beispiel 7 beschrieben kann man ausgehend von 18,2 g 1-[2-(4-Chlorphenyl)-ethyl]-piperazin und 17,5 g 2-Methoxy-5-cyano-benzoylchlorid das 1-[2-(4-Chlorphenyl)-ethyl]-4-(5-cyano-2-methoxy-benzyol)-piperazin vom Smp. 164-165° herstellen.

Beispiel 11: In analoger Weise wie in Beispiel 7 beschrieben kann man ausgehend von 22 g 1-[2-(4-Methoxyphenyl)-ethyl]-piperazin und 24 g 3,4-Dimethoxybenzyolchlorid das 1-[2-(4-Methoxyphenyl)-ethyl]-4-(3,4-dimethoxybenzoyl)-piperazin vom Smp. 120-120,5° herstellen.

Beispiel 12: In analoger Weise wie in Beispiel 7 beschrieben erhält man ausgehend von 26,9 g 1-[2-(4-Methoxyphenyl)-ethyl]-piperazin und 25 g 3-Methoxybenzoylchlorid das 1-[2-(4-Methoxyphenyl)-ethyl]-4-(3-methoxybenzoyl)-piperazin. Das Hydrochlorid schmilzt bei 188,5-191°.

Beispiel 13: In analoger Weise wie in Beispiel 7 beschrieben kann man ausgehend von 8,5 g 1-[2-(3-Chlorphenyl)-ethyl]-piperazin und 9,1 g 3,4-Dimethoxybenzyolchlorid das 1-[2-(3-Chlorphenyl)-ethyl]-4-(3,4-dimethoxybenzyol)-piperazin vom Smp. 116-117° herstellen.

Das Ausgangsmaterial kann z.B. folgendermassen hergestellt werden:

39,8 g 1-Ethoxycarbonyl-4-[2-(3-chlorphenyl)-ethyl)-piperazin, erhältlich durch Umsetzung von 17,7 g 1-Ethoxycarbonylpiperazin und 24,6 g 2-(3-Chlorphenyl)-ethylbromid unter Erwärmen auf 120-125° unter Stickstoff, werden mit 50 ml Aethanol, 50 g Kaliumhydroxid und 8,8 ml Wasser versetzt und 3 Stunden zum Rückfluss erhitzt. Man lässt abkühlen, verdünnt mit 300 ml Wasser und 300 ml Toluol, schüttelt gründlich durch, trennt die organische Phase ab, wäscht diese mit gesättigter Natriumchloridlösung, trochnet über Natriumsulfat und dampft unter vermindertem Druck zur Trockne ein. Man erhält das 1-[2-(3-Chlorphenyl)-ethyl)-piperazin, das über das Hydrochlorid (Smp. 260°) gereinigt und aus diesem durch Behandeln mit Natronlauge, Abtrennung mit Diethylether und Verdämpfer desselben wieder freigesetzt werden kann.

Beispiel 14: In analoger Weise wie in Beispiel 7 beschrieben kann man ausgehend von 7,2 g 1-[2-(3-Chlorphenyl)-ethyl)-piperazin und 6,54 g 3-Methoxybenzoylchlorid das 1-[2-(3-Chlorphenyl)-ethyl]-4-(3-methoxybenzoyl)-piperazin herstellen. Das Hydrochorid schmiltzt bei 197-198°.

Beispiel 15: In analoger Weise wie in Beispiel 7 beschrieben kann man ausgehend von 23,0 g 1-(2-Phenylethyl)-piperazin und 29,1 g 3,4-Dimethoxybenzoylchorid das 1-(2-Phenylethyl)-4-(3,4-dimethoxybenzyol)-piperazin vom Smp. 107,5-108,5° herstellen.

Beispiel 16: In analoger Weise wie in Beispiel 7 beschrieben kann man ausgehend von 25 g 1-(2-Phenylethyl)-piperazin und 23,9 g 3-Methoxybenzoylchorid das 1-(2-Phenylethyl)-4-(3-methoxybenzoyl)-piperazin herstellen. Das Hydrochlorid schmilzt bei 174-175°.

Beispiel 17: In analoger Weise wie in Beispiel 7 beschrieben kann man 5,3 g 1-[2-(4-Fluorphenyl)-ethyl]-piperazin und 6 g 3,4-Dimethoxybenzoylchlorid das 1-[2-(4-Fluorphenyl-ethyl]-4-(3,4-dimethoxybenzoyl)-piperazin herstellen. Das Hydrochlorid schmilzt bei 121-123°. Es enthält 1 Mol Kristallethanol.

Das Ausgangsmaterial kann z.B. folgendermassen hergestellt werden:
Zu einer Lösung von 50 g 2-(4-Fluorphenyl)-ethanol in 200 ml Toluol wird unter Eiskühlung innerhalb von 50 Minuten eine Lösung von 174,14 g Phosphortribromid in 80 ml Toluol zugetropft. Man lässt 5 Stunden bei Raumtemperatur nachrühren, giesst in 650 ml Eiswasser, trennt die organische Phase ab, schüttelt mit 500 ml Toluol nach, wäscht mit Wasser, trocknet über Natriumsulfat und dampft unter vermindertem Druck zur Trockne ein. Das rohe 2-(4-Fluorphenyl)-ethylbromid wird zur Reinigung in Chloroform gelöst und über 600 g Kieselgel filtriert.

27,85 g 2-(4-Fluorphenyl)-ethylbromid werden mit 21 ml 1-Ethoxycarbonylpiperazin vermischt und 3 Stunden bei 100° gerührt. Man lässt abkühlen, nimmt in 400 ml Chloroform auf, schüttelt mit 200 ml 2n-Natronlauge aus, trennt die organische Phase ab, trocknet über Natriumsulfat und verdampft das Chloroform. Das 1-Ethoxycarbonyl-4-[2-(4-Fluorphenyl)-ethyl]-piperazin bleibt als hellgelbes Oel zurück.

37,2 g 1-Ethoxycarbonyl-4-[2-(4-fluorphenyl)-ethyl]-piperazin werden in 55 ml Ethanol gelöst und unter Rühren tropfenweise mit einer Lösung von 47 g Kaliumhdroxid in 12,5 ml Wasser versetzt. Man erhitzt 6 Stunden unter Rühren auf 115-120°, dampft zur Trockne ein, nimmt mit Toluol auf, wäscht mit gesättigter Natriumchloridlösung und dampft unter vermindertem Druck zur Trockne ein. Das zurückbleibende 1-[2-(4-Fluorphenyl)-ethyl]-piperazin kann über das Dihydrochlorid vom Smp. 270-272° gereinigt werden.

Beispiel 18: In analoger Weise wie in Beispiel 17 beschrieben erhält man durch Umsetzung von 5,7 g 1-[2-(4-Fluorphenyl)-ethyl]-piperazin mit 5,7 g 4-Chlorbenzoylchlorid das 1-[2-(4-Fluorphenyl)-ethyl]-4-(4-chlorbenzyol)-piperazin. Das Hydrochlorid schmilzt bei 239-241°.

Beispiel 19: In analoger Weise wie in Beispiel 17 beschrieben erhält man durch Umsetzung von 7,2 g 4-Fluorbenzoylchlorid das 1-[2-(4-Fluorphenyl)-ethyl]-4-(4-fluorbenzoyl)-piperazin. Das Hydrochlorid schmilzt bei 218-225°.

Beispiel 20: In analoger Weise wie in Beispiel 17 beschrieben erhält man durch Umsetzung von 5,3 g 1-[2-(4-Fluorphenyl)-ethyl)-piperazinmit 5,1 g 3-Methoxybenzoylchlorid das 1-[2-(4-Fluorphenyl)-ethyl-4-(3-methoxybenzoyl)-piperazin. Das Hydrochlorid schmilzt bei 206-208°.

Beispiel 21: 5 g 1-[2-(4-Chlorphenyl)-ethyl]-piperazin werden in 75 ml Dichlormethan gelöst und innerhalb von 10 Minuten tropfenweise mit einer Lösung von 4,65 g 2-Trifluormethylbenzoylchlorid versetzt. Man erhitzt 5 Minuten zum Rückfluss, lässt 1 Stunde bei Raumtemperatur stehen, dampft zur Trockne ein, zeiht mit warmem Aceton aus und fällt mit Hexan. Man erhält das 1-[2-(4-Chlorphenyl)-ethyl]-4-(2-trifluormethylbenzoyl)-piperazin-hydrochlorid vom Smp. 212-214°.

Beispiel 22: In analoger Weise wie in Beispiel 18 beschrieben erhält man durch Umsetzung von 5 g 1-[2-(4-Chlorphenyl)-ethyl]-piperazin mit jeweils 4,65 g 3-Trifluormethylbenzoylchlorid bzw. 4-Trifluormethylbenzoylchlorid das 1-[2-(4-Chlorphenyl)-ethyl]-4-(3-trifluormethylbenzoyl)-piperazin-hydrochlorid, Smp. 233-235 (Zers.) und das 1-[2-(4-Chlorphenyl)-ethyl]-4-(4-trifluormethylbenzoyl)-piperazinhydrochlorid, Smp. 235-237°.

Beispiel 23: Eine bei Raumtemperatur gerührte Lösung von 22,4 g 1-[2-(4-Chlorphenyl)-ethyl]-piperazin und 12,1 g Triethylamin in 200 ml Methylenchlorid wird tropfenweise während 15 Minuten mit einer Lösung von 18,6 g p-Methylbenzoylchlorid in 30 ml Methylenchlorid versetzt. Das Reaktionsgemisch wird über Nacht bei Raumtemperatur gerührt. Die Reaktionslösung wird mit 100 ml Methylenchlorid verdünnt, mit 2n NaOH und Wasser gewaschen, über Natriumsulfat getrocknet und zur Trockne eingedampft. Der Rückstand wird zweimal aus Alkohol umkristallisiert. Man erhält so das 1-[2-(4-Chlorphenyl)-ethyl]-4-(4-methylbenzoyl)-piperazin vom Smp. 105-106°.

Beispiel 24: Eine bei Raumtemperatur gerührte Lösung von 6,6 g 1-[2-(4-Chlorphenyl)-ethyl]-piperazin in 70 ml Methylenchlorid wird tropfenweise während 10 Minuten mit einer Lösung von 4,7 g 2-Fluorobenzoylchlorid in 20 ml Methylenchlorid versetzt. Das Reaktionsgemisch wird über Nacht bei Raumtemperatur gerührt, mit ca. 250 ml Methylenchlorid verdünnt, mit Natronlauge und Wasser gewaschen, über Natriumsulfat getrocknet und zur Trockne eingedampft. Der Rückstand wird über das Hydrochlorid vom Smp. 233-235° gereinigt und charakterisiert. Man erhält so das 1-[2-(4-Chlorphenyl)-ethyl]-4-(2-fluorobenzoyl)-piperazin-hydrochlorid.

Beispiel 25: Eine bei Raumtemperatur gerührte Lösung von 13,4 g 1-[2-(4-Chlorphenyl)-ethyl]-piperazin in 100 ml Methylenchlorid wird tropfenweise während 10 Minuten mit einer Lösung von 4,7 g 2-Methylbenzoylchlorid in 50 ml Methylenchlorid versetzt. Das Reaktionsgemisch wird über Nacht bei Raumtemperatur gerührt. Die Suspension wird abgenutscht und mit eiskaltem Methylenchlorid/Ether-Gemisch nachgewaschen, getrocknet und als Hydrochlorid vom Smp. 234-235,5° charakterisiert. Man erhält so das 1-[2-(4-Chlorphenyl)-ethyl]4-(2-methylbenzoyl)piperazin-hydrochlorid.

Beispiel 26: Eine bei Raumtemperatur gerührte Lösung von 13,5 g 1-[2-(4-Chlorphenyl)-ethyl]-piperazin in 100 ml Methylenchlorid wird tropfenweise während 10 Minuten mit einer Lösung von 11,1 g 2,6-Dimethylbenzoylchlorid in 50 ml Methylenchlorid versetzt. Das Reaktionsgemisch wird über Nacht bei

Raumtemperatur gerührt. Die Suspension wird abgenutscht und mit eiskaltem Methylenchlorid und zuletzt mit Ether nachgewaschen, getrocknet und als Hydrochlorid vom Smp. 259-261° charakterisiert. Man erhält so das 1-[2-(4-Chlorphenyl)-ethyl]-4-(2,6-dimethylbenzoyl)piperazin-hydrochlorid.

Beispiel 27: Eine bei Raumtemperatur gerührte Lösung von 7,97 g 1-[2-(4-Chlorphenyl)-ethyl]-piperazin und 3,6 g Triethylamin in 150 ml Methylenchlorid wird tropfenweise während 10 Minuten mit einer Lösung von 6,51 g 3-Dimethylaminobenzoylchlorid in 130 ml Methylenchlorid versetzt. Das Reaktionsgemisch wird über Nacht bei Raumtemperatur gerührt, mit Methylenchlorid verdünnt, mit 2n NaOH alkalisch gestellt und mit Wasser gewaschen, über Natriumsulfat getrocknet und zur Trockne eingedampft. Der Rückstand wird aus Ethanol umkristallisiert. Man erhält so das 1-[2-(4-Chlorphenyl)-ethyl]-4-(3-dimethylaminobenzoyl)-piperazin vom Smp. 110-112°.

Das 3-Dimethylaminobenzoylchlorid wird folgendermassen hergestellt:
6,66 g 3-Dimethylaminobenzoesäure werden in 20,14 ml 2n KOH gelöst, abfiltriert und zur Trockne eingedampft. Die so erhaltenen Kristalle werden 6 Stunden bei 90 im Exsikkator getrocknet. Danach werden 7,85 g des Kaliumsalzes in 30 ml Benzol suspendiert und tropfenweise mit 4,9 g Oxalylchlorid in 10 ml Benzol versetzt. Man rührt 20 Minuten bei Raumtemperatur und kocht 1 Stunde am Rückfluss. Anschliessend wird das KCl abilftriert und die benzolische Lösung zur Trockne eingedampft. Man erhält so ein dunkelrotes Oel, welches sofort weiterumgesetzt wird.

Beispiel 28: Eine bei Raumtemperatur gerührte Lösung von 6,08 g 1-[2-(4-Chlorphenyl)-ethyl]-piperazin und 2,74 g Triethylamin in 150 ml Methylenchlorid wird tropfenweise während 10 Minuten mit einer Lösung von 5,1 g 4-Dimethylaminobenzoylchlorid in 130 ml Methylenchlorid versetzt. Das Reaktionsgemisch wird über Nacht bei Raumtemperatur gerührt, mit Methylenchlorid verdünnt, mit 2n NaOH alkalisch gestellt und mit Wasser gewaschen, über Natriumsulfat getrocknet und zur Trockne eingedampft. Der Rückstand wird aus Ethanol umkristallisiert. Man erhält so das 1-[2-(4-Chlorphenyl)-ethyl]-4-(4-dimethylaminobenzoyl)-piperazin vom Smp. 133-134°.

Das 4-Dimethylaminobenzoylchlorid wird folgendermassen hergestellt:
6,66 g 3-Dimethylaminobenzoesäure werden in 20,14 ml 2n KOH gelöst, abfiltriert und zur Trockne eingedampft. Die so erhaltenen Kristalle werden 6 Stunden bei 90 im Exsikkator getrocknet. Danach werden 5,5 g des Kaliumsalzes in 40 ml Benzol suspendiert und tropfenweise mit 3,4 g Oxalychlorid in 15 ml Benzol versetzt. Man rührt 20 Minuten bei Raumtemperatur und kocht 1 Stunde am Rückfluss. Anschliessend wird das KCl abilftriert und die benzolische Lösung zur Trockne eingedampft. Man erhält gelbe Kristalle, welche sofort weiterumgesetzt werden.

Beispiel 29: Eine bei Raumtemperatur gerührt Lösung von 8,73 g 1-[2-(4-Chlorphenyl)-ethyl]-piperazin in 50 ml Dimethylformamid wird tropfenweise während 10 Minuten mit einer Lösung von 4-Acetaminobenzoesäureanhydrid (aus 6,97 g 4-Acetaminobenzoesäure in 70 ml DMF, 3,93 g Triethylamin und 4,22 g Chlorameisensäureethylester in 30 ml Chloroform bei 0-5° hergestellt) über Nacht bei Raumtemperatur gerührt. Die Reaktionslösung wird zur Trockne eingedampft und der Rückstand in Methylenchlorid gelöst, mit Sodalösung alkalisch gestellt und mit Wasser neutral gewaschen, über Natriumsulfat getrocknet und eingedampft. Das Rohprodukt wird aus Ethanol und Ether umkristallisiert. Man erhält so das 1-[2-(4-Chlorphenyl)-ethyl]-4-(4-acetaminobenzoyl)piperazin vom Smp. 147-149°.

Beispiel 30: Die Lösung von 80,5 g 1-(p-Chlorphenethyl)piperazin in 1200 ml $CH_2Cl_2$ wird bei Raumtemperatur mit 71,9 g 3,5-Dimethoxybenzoylchlorid gelöst in 700 ml $CH_2Cl_2$ versetzt. Die Zugaberate wird so gewählt, dass die exatherme Reaktion unter Kontrolle bleibt. Anschliessend wird 15 Minuten am Rückfluss und noch 1 Stunde bei Raumtemperatur gerührt. Nach der Zugabe von 1900 ml Diethyläther wird 15 Minuten gerührt, und der entstandene Niederschlag abgesaugt. Der Niederschlag wird in 3,3 l siedendem Ethanol gelöst. Der nach dem Abkühlen auf ca. 20° anfallende farblose Niederschlag wird abgesaugt, mit wenig Ethanol gewaschen und im Hochvakuum bei 80° während 16 Studen getrocknet. Man erhält so 1-p-Chlorphenethyl-4-(3,5-dimethoxybenzoyl)-piperazin-hydrochlorid vom Smp. 244-246° (Zers.).

Das Ausgangsmaterial kann wie folgt hergestellt werden:
72,9 g 3,5-Dimethoxybenzoesäure werden in 320 ml Toluol suspendiert und mit 2 Tropfen Dimethylformamid versetzt. Die Suspension wird auf 50° erwärmt. Bei dieser Temperatur werden 40 ml Thionylchlorid innert 10 Minuten zugetropft. Dann wird auf 90° erwärmt (heftige Gasentwicklung) und noch 2 Stunden gerührt. Das Reaktionsgemisch wird abgekühlt und am Rotationsverdampfer eingedampft. Nach der Zugabe von Toluol wird erneut eingedampft. Der ölige Rückstand wird noch 15 Minuten am Hochvakuum getrocknet. Man erhält so 3,5-Dimethoxybenzoylchlorid.

Beispiel 31: In analoger Weise wie in Beispiel 30 beschrieben erhält man:
aus 3,5-Dichlorbenzoylchlorid das
1-(4-Chlorphenethyl)-4-(3,5-dichlorbenzoyl)-piperazin-hydrochlorid vom Smp. 239-241°,
aus 2,6-Dimethoxybenzoylchlorid das

1-(4-Chlorphenethyl)-4-(2,6-dimethoxyenzoyl)-piperazin-hydrochlorid vom Smp. 243-244° (Zers.) (kristallisiert aus Methylenhlorid/Hexan),
aus 3,5-Dimethylbenzoylchlorid das
1-(4-Chlorphenethyl)-4-(3,5-dimethylbenzoyl)-piperazin-hydrochlorid vom Smp. 228-231° (Zers.) (kristallisiert aus Aethanol).

Beispiel 32: Eine bei Raumtemperatur gerührt Lösung von 5 g 1-(4-Chlorphenethyl)-piperazin in 75 ml Methylenchlorid wird tropfenweise während 10 Minuten mit einer Lösung von 4,65 g 2-Trifluoromethylbenzoylchlorid in 50 ml Methylenchlorid versetzt. Das Reaktionsgemisch wird 5 Minuten zum Rückfluss erhitzt, 1 Stunde bei Raumtemperatur stehen gelassen und eingedampft. Der Rückstand wird in heissem Aceton aufgeschlämmt und nach der Zugabe von Hexan filtriert. Man erhält 1-(4-Chlorphenethyl)-4-(2-trifluoromethylbenzoyl)-piperazin-hydrochlorid vom Smp. 212-214°.

Beispiel 33: 10 g 3-Acetamidobenzoesäure werden in 100 ml DMF gelöst. Bei einer Temperatur von -5° bis 0° werden unter Rühren zuerst 5,65 g Triethylamin und dann 6,05 g in 40 ml $CH_2Cl_2$ gelöster Chlorameisensäureethylester zugetropft. Man rührt noch 30 Minuten bei 0° bis +5° nach, und bei der gleichen Temperatur tropft man 12,54 g 1-(p-Chlorphenethyl)-piperazin in 75 ml DMF zu. Ueber Nacht wird bei Raumtemperatur gerührt und anschliessend zur Trockne eingedampft. Der Rückstand wird in 350 ml $Ch_2Cl_2$ aufgenommen und mit 150 ml 2N NaOH und 50 ml $H_2O$ gewaschen und über $Na_2SO_4$ getrocknet. Nach dem Abfiltrieren und Eindampfen gewinnt man ein braunes Oel, welches mit Ether zur Kristallisation gebracht wird. Aus Ethanol/Ether wird umkristallisiert. Man erhält so 1-[2-(p-Chlorphenyl)-ethyl]-4-(3-acetamidobenzoyl)-piperazin vom Smp. 101-7°.

Beispiel 34: 3,42 g Benzylpiperazin werden in 35 ml Methylenchlorid gelöst, mit 2,1 g Triethylamin und dann tropfenweise unter Eiskühlung mit 3,4 g 3-Methoxybenzoylchlorid in 30 ml Methylenchlorid versetzt. Die Reaktionsmischung wird über Nacht bei RT gerührt und dann mit $H_2O$ gewaschen. Nach dem Trocknen über Natriumsulfat wird die Lösung im Vakuum zur Trockne eingedampft. Das ölige 1-Benzyl-4-(3-methoxybenzoyl)-piperazin wird in 150 ml Feinsprit gelöst und über 0,5 g Pd-C debenzyliert. Nach Beendigung der Hydrierung wird die Mischung vom Katalysator abfiltriert und im Vakuum zur Trockne eingedampft. Das so erhaltene ölige N-(3-Methoxybenzoyl)-piperazin wird mit 3,8 g p-Chlorphenethylbromid in einem Sulfierkolben vorgelegt und auf 100° geheizt. Bei 60° findet eine leichte exotherme Reaktion statt. Nach 3h bei 100° wird die Mischung abgekühlt und in 400 ml Methylenchlorid gelöst, mit 2N NaOH versetzt, ausgeschüttelt, und die Methylenchlorid-Phase wird 2-mal mit $H_2O$ gewaschen. Die organische Phase wird über $Na_2SO_4$ getrocknet und im Vakuum zur Trockne eingedampft. Das ölige 1-[2-(4-Chlorphenyl)-ethyl]-4-(3-methoxybenzoyl)-piperazin wird in Isopropanol gelöst und mit Salzsäure versetzt. Nach Zugabe von Ether kristallisiert das Hydrochlorid vom F. 230-232°.

Beispiel 35: 36,05 g 1-[2-(4-Chlorphenyl)-ethyl]-4-(3,5-dihydroxybenzoyl)-piperazin in 140 ml 10%iger Kalilauge werden vorsichtig mit 25,2 g Dimethylsulfat versetzt. Das Reaktionsgemisch wird noch 30 Minuten auf 100° erwärmt, und mit $CH_2Cl_2$ extrahiert. Die vereinigten organischen Extrakte werden über $MgSO_4$ getrocknet und eingedampft. Das ölige 1-[2-(4-Chlorphenyl)-ethyl]-4-(3,5-dimethoxybenzoyl)-piperazin wird über das Hydrochlorid vom Smp. 244-246° (Zers.) gereinigt.

Das Ausgangsmaterial kann z.B. folgendermassen hergestellt werden:
50 g 3,5-Dihydroxybenzoesäure werden in 132,2 g Acetanhydrid suspendiert und auf 50° erwärmt. Nach Zugabe von 15 Tropfen konz. $H_2SO_4$ wird die nun entstandene Lösung 1 Stunde bei 60° gerührt. Das Reaktionsgemisch wird auf Eis gegossen und mit $CH_2Cl_2$ extrahiert. Die vereinigten organischen Phasen werden 3 x mit $H_2O$ gewaschen, über $Na_2SO_4$ getrocknet und eingedampft. Der Rückstand wird aus Ether kristallisiert und ergibt die 3,5-Diacetoxybenzoesäure vom Smp. 140-145°.

42 g 3,5-Diacetoxybenzoesäure werden mit 300 ml Benzol und 26 ml Thionylchlorid während 5 Stunden zum Rückfluss erhitzt. Das Reaktionsgemisch wird eingedampft, der Rückstand in Benzol aufgenommen und erneut eingedampft. Man erhält so das 3,5-Diacetoxybenzoylchlorid vom Smp. 85-87°.

In analoger Weise wie in Beispiel 1 beschrieben erhält man aus 3,5-Diacetoxybenzoylchlorid das 1-[2-(4-Chlorphenyl)-ethyl]-4-(3,5-diacetoxybenzoyl)-piperazin-hydrochlorid vom Smp. 220° (Zers.).

Die Lösung von 1-[2-(4-Chlorphenyl)-ethyl]-4-(3,5-diacetoxybenzoyl)-piperazin in 500 ml Methanol wird mit 285 ml 1N NaOH während 16 Stunden bei 20° gerührt. Das Reaktionsgemisch wird mit $H_2O$ verdünnt und mit Essigsäure auf pH 6 gestellt. Der ausgefallene ölige Niederschlag wird mit $CH_2Cl_2$ verrührt und abgesaugt. Kristallisation aus Ethanol ergibt 1-[2-(4-Chlorphenyl)-ethyl]-4-(3,5-dihydroxybenzoyl)-piperazin vom Smp. 217 - 221°.

Beispiel 36: Eine Mischung von 5 g 1-(4-Chlorphenethyl)-piperazin, 9,25 g wasserfreiem Kaliumcarbonat, 4,34 g 3-Trifluormethylbenzylchlorid und 100 ml absolutem Alkohol wird unter Rühren während 15 Stunden zum Rückfluss erhitzt und unter vermindertem Druck eingedampft. Der Rückstand wird in Wasser aufgenommen und dreimal mit je 70 ml Methylenchlorid extrahiert. Trocknen der vereinigten organischen

Phasen über Magnesiumsulfat und erneutes Einengen ergibt ein gelbes Oel, das in 70 ml Essigsäureethylester gelöst wird. Das durch Zugabe einer Lösung von Chlorwasserstoff in Essigsäurethylester ausgefallene 1-(4-Chlorphenethyl)-4-(3-trifluoromethylbenzyl)-piperazin-dihydrochlorid wird abgesaugt und mit Ether gewaschen. Zur weiteren Reinigung wird in heissem Aceton aufgeschlämmt und nach Verdünnen mit Hexan filtriert. Smp. 254-255° (Zers.).

Beispiel 37: Analog Beispiel 36 werden die folgenden Verbindungen hergestellt:
aus 2-Fluorbenzylchlorid das 1-(4-Chlorphenethyl)-4-(2-fluorbenzyl)-piperazin-dihydrochlorid vom Smp. 248-249° (Zers.),
aus 4-Fluorbenzylchlorid das 1-(4-Chlorphenethyl)-4-(4-fluorbenzyl)-piperazin-dihydrochlorid vom Smp. 265-268° (Zers.),
aus 2-Chlorbenzylchlorid das 1-(4-Chlorphenethyl)-4-(2-chlorbenzyl)-piperazin-dihydrochlorid vom Smp. ab 240° (Zers.),
aus 3-Chlorbenzylchlorid das 1-(4-Chlorphenethyl)-4-(3-chlorbenzyl)-piperazin-dihydrochlorid vom Smp. ab 260° (Zers.),
aus 4-Chlorbenzylchlorid das 1-(4-Chlorphenethyl)-4-(4-chlorbenzyl)-piperazin-dihydrochlorid vom Smp. ab 250° (langsame Zers.),
aus 2,4-Dichlorbenzylchlorid das 1-(4-Chlorphenethyl)-4-(2,4-dichlorbenzyl)-piperazin-hydrochlorid vom Smp. ab 250° (Zers.).

Beispiel 38: Tabletten enthaltend 25 mg Wirkstoff, z.B. 1-[2-(4-Chlorphenyl)-ethyl]-4-(3,5-dimethoxybenzoyl)-piperazin, können folgendermassen hergestellt werden:

| Bestandteile (für 1000 Tabletten) | |
|---|---|
| Wirkstoff | 25,0 g |
| Lactose | 100,7 g |
| Weizenstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung

Sämtliche festen Ingredienzen werden zunächst durch ein Sieb von 0,6 mm Maschenweite getrieben. Dann wird die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben, mit der Pulvermischung zu einer knetbaren Masse verarbeitet und das erhaltene Gemisch, wenn nötig unter Hinzufügen von Wasser, granuliert. Das Granulat wird über nacht bei 35° getrocknet, durch ein Sieb mit 1,2 mm Maschenweite getrieben und zu beidseitig konkaven Tabletten von etwa 6 mm Durchmesser verpresst.

Beispiel 39: In analoger Weise wie in Beispiel 38 beschrieben, können auch Tabletten, enthaltend jeweils 25 mg einer anderen der in den Beispielen 1 bis 37 genannten Verbindungen hergestellt werden.

**Patentansprüche**
**Patentansprüche für folgende Vertragsstaaten : BE, CH, DE, FR, GB, IT, LI, LU, NL, SE**

**1.** Piperazine der Formel

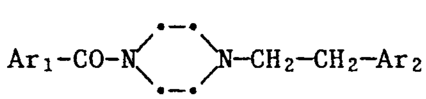

(I)

und ihre Salze, worin $Ar_1$ unsubstituiertes oder ein- oder zweifach durch $C_1$-$C_7$-Alkyl, $C_1$-$C_7$-Alkoxy, Cyano, Halogen, Trifluormethyl, Amino, $C_1$-$C_7$-Alkylamino, Di-$C_1$-$C_7$-alkylamino und/oder $C_1$-$C_7$-Alkanoylamino substituiertes Phenyl bedeutet, und worin $Ar_2$ unsubstituiertes oder ein- oder zweifach durch $C_1$-$C_7$-Alkyl, $C_1$-$C_7$-Alkoxy oder Halogen substituiertes Phenyl bedeutet.

**2.** Verbindungen gemäss Anspruch 1 der Formel I und ihre Salze, worin $Ar_1$ durch Halogen mit Atomnummer bis und mit 35, durch Trifluormethyl, durch Di-$C_1$-$C_4$-alkylamino oder durch $C_2$-$C_5$-Alkanoylamino jeweils monosubstituiertes Phenyl oder durch $C_1$-$C_4$-Alkyl oder durch $C_1$-$C_4$-Alkoxy jeweils disubstituiertes Phenyl bedeutet und $Ar_2$ durch Halogen mit Atomnummer bis und mit 35 monosubstituiertes Phenyl bedeutet.

**3.** Verbindungen gemäss Anspruch 1 der Formel I und ihre Salze, worin $Ar_1$ unsubstituiertes oder ein- oder zweifach durch $C_1$-$C_4$-Alkyl, $C_1$-$C_4$-Alkoxy, Halogen, Trifluormethyl, Di-$C_1$-$C_4$-alkylamino und/oder $C_1$-$C_5$-Alkanoylamino substituiertes Phenyl bedeutet und $Ar_2$ unsubstituiertes oder ein- oder zweifach durch $C_1$-$C_4$-Alkoxy oder Halogen substituiertes Phenyl bedeutet.

**4.** Verbindungen gemäss Anspruch 1 der Formel I und ihre Salze, worin $Ar_1$ unsubstituiertes oder ein- oder zweifach durch $C_1$-$C_4$-Alkyl, $C_1$-$C_4$-Alkoxy, Halogen und/oder Trifluormethyl substituiertes Phenyl bedeutet und $Ar_2$ durch Halogen monosubstituiertes Phenyl bedeutet.

**5.** Verbindungen gemäss Anspruch 1 der Formel I und ihre Salze, worin $Ar_1$ unsubstituiertes oder ein- oder zweifach durch $C_1$-$C_4$-Alkyl, $C_1$-$C_4$-Alkoxy, Halogen und/oder Trifluormethyl substituiertes Phenyl bedeutet und $Ar_2$ durch $C_1$-$C_4$-Alkoxy monosubstituiertes Phenyl bedeutet.

**6.** Verbindungen gemäss Anspruch 1 der Formel I und ihre Salze, worin $Ar_1$ 3,5-Di-$C_1$-$C_4$-alkoxy-phenyl bedeutet und $Ar_2$ 4-Halogenphenyl bedeutet.

**7.** Verbindungen gemäss Anspruch 1 der Formel I und ihre Salze, worin $Ar_1$ 2- oder 4-Fluor-, 2- oder 4-Chlor-, 2- oder 3-Trifluormethyl-, 3- oder 4-Dimethylamino-, 3- oder 4-Acetylamino-, 3,4- oder 3,5-Dimethoxy- oder 2,6-Dimethylphenyl bedeutet und $Ar_2$ 4-Fluor- oder 4-Chlorphenyl bedeutet.

**8.** Verbindungen gemäss Anspruch 1 der Formel I und ihre Salze, worin $Ar_1$ 2- oder 4-Fluor-, 3-Trifluormethyl-, 4-Chlor-, 3- oder 4-Dimethylamino- oder 3- oder 4-Acetylamino-phenyl bedeutet und $Ar_2$ 4-Fluor- oder 4-Chlorphenyl bedeutet.

**9.** Verbindungen gemäss Anspruch 1 der Formel I und ihre Salze, worin $Ar_1$ durch Methyl, Methoxy, Fluor, Chlor oder Trifluormethyl monosubstituiertes oder durch Methoxy disubstituiertes Phenyl bedeutet und $Ar_2$ durch Fluor oder Chlor monosubstituiertes Phenyl bedeutet.

**10.** Verbindungen gemäss Anspruch 1 der Formel I und ihre Salze, worin $Ar_1$ durch Methyl oder Fluor monosubstituiertes Phenyl bedeutet und $Ar_2$ durch Fluor oder Chlor monosubstituiertes Phenyl bedeutet.

**11.** Verbindungen gemäss Anspruch 1 der Formel I und ihre Salze, worin $Ar_1$ 2- oder 4-Fluorphenyl, 2-Methylphenyl oder 3,5-Dimethoxyphenyl bedeutet und $Ar_2$ 4-Fluor- oder 4-Chlorphenyl bedeutet.

**12.** 1-[2-(4-Chlorphenyl)-ethyl]-4-(4-chlorbenzoyl)-piperazin,
1-[2-(4-Chlorphenyl)-ethyl]-4-(3-chlorbenzoyl)-piperazin,
1-[2-((4-Chlorphenyl)-ethyl]-4-(2-chlorbenzoyl)-piperazin,
1-[2-(4-Chlorphenyl)-ethyl]-4-(2,6-dichlorbenzoyl)-piperazin,
1-[2-(4-Chlorphenyl)-ethyl]-4-(2-fluorbenzoyl)-piperazin,
1-[2-(4-Chlorphenyl)-ethyl]-4-(2,4-dichlorbenzoyl)-piperazin,
1-[2-(4-Chlorphenyl)-ethyl]-4-(4-fluorbenzoyl)-piperazin,
1-[2-(4-Chlorphenyl)-ethyl]-4-(3,4-dichlorbenzoyl)-piperazin,
1-[2-(4-Chlorphenyl)-ethyl]-4-(3-fluorbenzoyl)-piperazin,
1-[2-(4-Chlorphenyl)-ethyl]-4-(2-aminobenzoyl)-piperazin,
1-(2-Phenylethyl)-4-(2-amino-benzoyl)-piperazin,
1-[2-(4-Chlorphenyl)-ethyl]-4-(3-methoxy-benzoyl)-piperazin,
1-[2-(4-Chlorphenyl)-ethyl]-4-(3,4-dimethoxybenzoyl)-piperazin,
1-[2-(4-Chlorphenyl)-ethyl]-4-(4-methoxybenzoyl)-piperazin,
1-[2-(4-Chlorphenyl)-ethyl]-4-(4-methylbenzoyl)-piperazin,
1-[2-(4-Chlorphenyl)-ethyl]-4-(2-methoxybenzoyl)-piperazin,
1-[2-(4-Chlorphenyl)-ethyl]-4-(5-cyano-2-methoxy-benzoyl)-piperazin,

1-[2-(4-Methoxyphenyl)-ethyl]-4-(3,4-dimethoxybenzoyl)-piperazin,
1-[2-(4-Methoxyphenyl)-ethyl]-4-(3-methoxybenzoyl)-piperazin,
1-[2-(3-Chlorphenyl)-ethyl]-4-(3,4-dimethoxybenzoyl)-piperazin,
1-[2-(3-Chlorphenyl)-ethyl]-4-(3-methoxybenzoyl)-piperazin,
1-(2-Phenylethyl)-4-(3,4-dimethoxybenzoyl)-piperazin,
1-(2-Phenylethyl)-4-(3-methoxybenzoyl)-piperazin,
1-[2-(4-Fluorphenyl)-ethyl]-4-(3,4-dimethoxybenzoyl)-piperazin,
1-[2-(4-Fluorphenyl)-ethyl]-4-(4-chlorbenzoyl)-piperazin,
1-[2-(4-Fluorphenyl)-ethyl]-4-(4-fluorbenzoyl)-piperazin,
1-[2-(4-Fluorphenyl)-ethyl-4-(3-methoxybenzoyl)-piperazin,
1-[2-(4-Chlorphenyl)-ethyl]-4-(2-trifluormethylbenzoyl)-piperazin,
1-[2-(4-Chlorphenyl)-ethyl]-4-(3-trifluormethylbenzoyl)-piperazin,
1-[2-(4-Chlorphenyl)-ethyl]-4-(4-trifluormethylbenzoyl)-piperazin,
1-[2-(4-Chlorphenyl)-ethyl]-4-(4-methylbenzoyl)-piperazin,
1-[2-(4-Chlorphenyl)-ethyl]-4-(2-methylbenzoyl)-piperazin,
1-[2-(4-Chlorphenyl)-ethyl]-4-(2,6-dimethylbenzoyl)-piperazin,
1-[2-(4-Chlorphenyl)-ethyl]-4-(3,5-dichlorbenzoyl)-piperazin,
1-[2-(4-Chlorphenyl)-ethyl]-4-(2,6-dimethoxybenzoyl)-piperazin
oder ein Salz davon.

**13.** 1-[2-(4-Chlorphenyl)-ethyl]-4-(2-fluorobenzoyl)piperazin oder ein Salz davon.

**14.** 1-[2-(4-Chlorphenyl)-ethyl]-4-(3-dimethylaminobenzoyl)piperazin oder ein Salz davon.

**15.** 1-[2-(4-Chlorphenyl)-ethyl]-4-(4-dimethylaminobenzoyl)piperazin oder ein Salz davon.

**16.** 1-[2-(4-Chlorphenyl)-ethyl]-4-(4-acetaminobenzoyl)piperazin oder ein Salz davon.

**17.** 1-[2-(4-Chlorphenyl)-ethyl]-4-(3,5-dimethoxybenzoyl)piperazin oder ein Salz davon.

**18.** 1-[2-(p-Chlorphenyl)-ethyl]-4-(3-acetamidobenzoyl)piperazin oder ein Salz davon.

**19.** Verbindung gemäss einem der Ansprüche 1-18 zur Anwendung in einem Verfahren zur therapeutischen oder prophylaktischen Behandlung des menschlichen oder tierischen Körpers.

**20.** Verbindung gemäss einem der Ansprüche 1-18 zur Verwendung als Analgetikum.

**21.** Pharmazeutische Präparate enthaltend eine Verbindung gemäss einem der Ansprüche 1-18 in freier Form oder in Form eines pharmazeutisch verwendbaren Salzes zusammen mit einem pharmazeutisch anwendbaren Trägermaterial.

**22.** Verwendung von Verbindungen gemäss einem der Ansprüche 1-18 zur Herstellung von Analgetika.

**23.** Verfahren zur Herstellung von Verbindungen gemäss einem der Ansprüche 1-18, dadurch gekennzeichnet, dass man

a) eine Verbindung der Formel

$Ar_1$-$X_1$ (IIa)

oder ein Salz davon, worin $X_1$ Carboxy oder reaktionsfähiges funktionell abgewandeltes Carboxy bedeutet, mit einer Verbindung der Formel

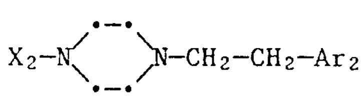 (IIb)

oder einem Salz davon umsetzt, worin $X_2$ Wasserstoff oder eine Aminoschutzgruppe bedeutet, oder

b) eine Verbindung der Formel

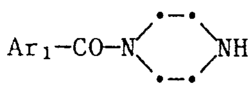 (IIIa)

oder ein Salz davon mit einer Verbindung der Formel

$X_3$-$CH_2$-$CH_2$-$Ar_2$ (IIIb)

oder einem Salz davon umsetzt, worin $X_3$ Hydroxy oder reaktionsfähiges verestertes Hydroxy bedeutet, oder

c) eine Verbindung der Formel

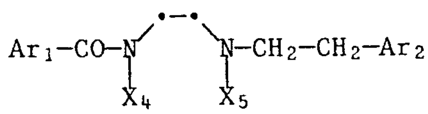 (IV)

oder ein Salz davon, worin einer der Reste $X_4$ und $X_5$ Wasserstoff ist und der andere für eine Gruppe der Formel -$CH_2$-$CH_2$-$X_3$ steht, und $X_3$ Hydroxy oder reaktionsfähiges verestertes Hydroxy bedeutet, intramolekular kondensiert oder

d) eine Verbindung der Formel

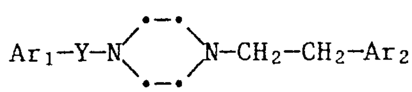 (V)

oder ein Salz davon, worin Y eine zu -CO- oxidierbare Gruppe bedeutet, oxidiert,

und gewünschtenfalls eine verfahrensgemäss oder auf andere Weise erhältliche Verbindung in eine andere Verbindung der Formel I überführt, ein verfahrensgemäss erhältliches Isomerengemisch in die Komponenten auftrennt, eine verfahrensgemäss erhältliche freie Verbindung der Formel I in ein Salz überführt und/oder ein verfahrensmäss erhältliches Salz in die freie Verbindung der Formel I oder in ein anderes Salz überführt.

**24.** Verfahren zur Herstellung von Verbindungen der Formel I gemäss einem der Ansprüche 1-18 und ihrer Salze, worin $Ar_1$ durch $C_1$-$C_7$-Alkoxy mono- oder disubstituiertes Phenyl bedeutet, dadurch gekennzeichnet, dass man eine Verbindung der Formel

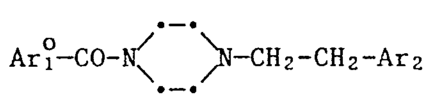 (VI)

oder ein Salz davon, worin $Ar_1^o$ durch Hydroxy mono- oder disubstituiertes Phenyl bedeutet, alkyliert und gewünschtenfalls eine verfahrensgemäss oder auf andere Weise erhältliche Verbindung in eine andere Verbindung der Formel I überführt, ein verfahrensgemäss erhältliches Isomerengemisch in die Komponenten auftrennt, eine verfahrensgemäss erhältliche freie Verbindung der Formel I in ein Salz überführt und/oder ein verfahrensmäss erhältliches Salz in die freie Verbindung der Formel I oder in ein anderes Salz überführt.

**25.** Verfahren zur Herstellung pharmazeutischer Präparate gemäss Anspruch 21, dadurch gekennzeichnet, dass man eine Verbindung gemäss einem der Ansprüche 1-18 freier Form oder in Form eines pharmazeutisch verwendbaren Salzes, gegebenenfalls unter Beimischung von üblichen Hilfs- und Trägerstoffen, zu pharmazeutischen Präparaten verarbeitet.

**26.** Verbindungen der Formel

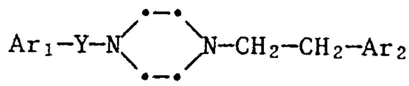

(V)

und ihre Salze, worin Y für $-CH_2-$ steht und $Ar_1$ und $Ar_2$ die gemäss einem der Ansprüche 1-11 angegebenen Bedeutungen haben.

**27.** Verwendung von Verbindungen gemäss Anspruch 26 zur Herstellung von Analgetika.

**Patentansprüche für folgende Vertragsstaaten : AT, ES, GR**

**1.** Verfahren zur Herstellung von Piperazinen der Formel

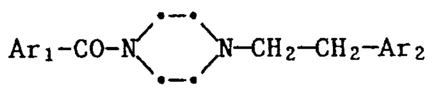

(I)

und ihren Salzen, worin $Ar_1$ unsubstituiertes oder ein- oder zweifach durch $C_1$-$C_7$-Alkyl, $C_1$-$C_7$-Alkoxy, Cyano, Halogen, Trifluormethyl, Amino, $C_1$-$C_7$-Alkylamino, Di-$C_1$-$C_7$-alkylamino und/oder $C_1$-$C_7$-Alkanoylamino substituiertes Phenyl bedeutet, und worin $Ar_2$ unsubstituiertes oder ein- oder zweifach durch $C_1$-$C_7$-Alkyl, $C_1$-$C_7$-Alkoxy oder Halogen substituiertes Phenyl bedeutet, dadurch gekennzeichnet, dass man

a) eine Verbindung der Formel

$Ar_1$-$X_1$ (IIa)

oder ein Salz davon, worin $X_1$ Carboxy oder reaktionsfähiges funktionell abgewandeltes Carboxy bedeutet, mit einer Verbindung der Formel

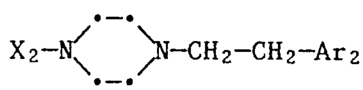

(IIb)

oder einem Salz davon umsetzt, worin $X_2$ Wasserstoff oder eine Aminoschutzgruppe bedeutet, oder

b) eine Verbindung der Formel

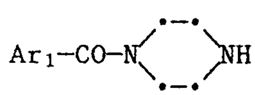

(IIIa)

oder ein Salz davon mit einer Verbindung der Formel

$X_3$-$CH_2$-$CH_2$-$Ar_2$ (IIIb)

oder einem Salz davon umsetzt, worin $X_3$ Hydroxy oder reaktionsfähiges verestertes Hydroxy bedeutet, oder

c) eine Verbindung der Formel

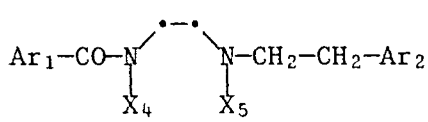

(IV)

oder ein Salz davon, worin einer der Reste $X_4$ und $X_5$ Wasserstoff ist und der andere für eine Gruppe der Formel $-CH_2-CH_2-X_3$ steht, und $X_3$ Hydroxy oder reaktionsfähiges verestertes Hydroxy bedeutet, intramolekular kondensiert oder

d) eine Verbindung der Formel

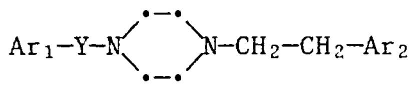

(V)

oder ein Salz davon, worin Y eine zu -CO- oxidierbare Gruppe bedeutet, oxidiert,

und gewünschtenfalls eine verfahrensgemäss oder auf andere Weise erhältliche Verbindung in eine andere Verbindung der Formel I überführt, ein verfahrensgemäss erhältliches Isomerengemisch in die Komponenten auftrennt, eine verfahrensgemäss erhältliche freie Verbindung der Formel I in ein Salz überführt und/oder ein verfahrensmäss erhältliches Salz in die freie Verbindung der Formel I oder in ein anderes Salz überführt.

**2.** Verfahren zur Herstellung von Verbindungen der in Anspruch 1 wiedergegebenen Formel I und ihrer Salze, worin $Ar_1$ durch $C_1$-$C_7$-Alkoxy mono- oder disubstituiertes Phenyl bedeutet, dadurch gekennzeichnet, dass man eine Verbindung der Formel

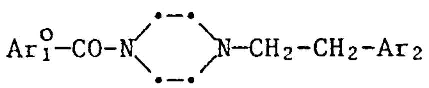

(VI)

oder ein Salz davon, worin $Ar_1^o$ durch Hydroxy mono- oder disubstituiertes Phenyl bedeutet, alkyliert und gewünschtenfalls eine verfahrensgemäss oder auf andere Weise erhältliche Verbindung in eine andere Verbindung der Formel I überführt, ein verfahrensgemäss erhältliches Isomerengemisch in die Komponenten auftrennt, eine verfahrensgemäss erhältliche freie Verbindung der Formel I in ein Salz überführt und/oder ein verfahrensmäss erhältliches Salz in die freie Verbindung der Formel I oder in ein anderes Salz überführt.

**3.** Verfahren gemäss Anspruch 1 oder 2, dadurch gekennzeichnet, dass man Verbindungen der Formel I und ihre Salze, worin $Ar_1$ durch Halogen mit Atomnummer bis und mit 35, durch Trifluormethyl, durch Di-$C_1$-$C_4$-alkylamino oder durch $C_2$-$C_5$-Alkanoylamino jeweils monosubstituiertes Phenyl oder durch $C_1$-$C_4$-Alkyl oder durch $C_1$-$C_4$-Alkoxy jeweils disubstituiertes Phenyl bedeutet und $Ar_2$ durch Halogen mit Atomnummer bis und mit 35 monosubstituiertes Phenyl bedeutet, herstellt.

**4.** Verfahren gemäss Anspruch 1 oder 2, dadurch gekennzeichnet, dass man Verbindungen der Formel I und ihre Salze, worin $Ar_1$ unsubstituiertes oder ein- oder zweifach durch $C_1$-$C_4$-Alkyl, $C_1$-$C_4$-Alkoxy, Halogen, Trifluormethyl, Di-$C_1$-$C_4$-alkylamino und/oder $C_1$-$C_5$-Alkanoylamino substituiertes Phenyl bedeutet und $Ar_2$ unsubstituiertes oder ein- oder zweifach durch $C_1$-$C_4$-Alkoxy oder Halogen substituiertes Phenyl bedeutet, herstellt.

**5.** Verfahren gemäss Anspruch 1 oder 2, dadurch gekennzeichnet, dass man Verbindungen der Formel I und ihre Salze, worin $Ar_1$ unsubstituiertes oder ein- oder zweifach durch $C_1$-$C_4$-Alkyl, $C_1$-$C_4$-Alkoxy, Halogen und/oder Trifluormethyl substituiertes Phenyl bedeutet und $Ar_2$ durch Halogen monosubstituiertes Phenyl bedeutet, herstellt.

**6.** Verfahren gemäss Anspruch 1 oder 2, dadurch gekennzeichnet, dass man Verbindungen der Formel I und ihre Salze, worin $Ar_1$ unsubstituiertes oder ein- oder zweifach durch $C_1$-$C_4$-Alkyl, $C_1$-$C_4$-Alkoxy, Halogen und/oder Trifluormethyl substituiertes Phenyl bedeutet und $Ar_2$ durch $C_1$-$C_4$-Alkoxy monosubstituiertes Phenyl bedeutet, herstellt.

**7.** Verfahren gemäss Anspruch 1 oder 2, dadurch gekennzeichnet, dass man Verbindungen der Formel I und ihre Salze, worin $Ar_1$ 3,5-Di-$C_1$-$C_4$-alkoxy-phenyl bedeutet und $Ar_2$ 4-Halogenphenyl bedeutet, herstellt.

**8.** Verfahren gemäss Anspruch 1 oder 2, dadurch gekennzeichnet, dass man Verbindungen der Formel I und ihre Salze, worin $Ar_1$ 2- oder 4-Fluor-, 2- oder 4-Chlor-, 2- oder 3-Trifluormethyl-, 3- oder 4-Dimethylamino-, 3- oder 4-Acetylamino-, 3,4- oder 3,5-Dimethoxy- oder 2,6-Dimethylphenyl bedeutet und $Ar_2$ 4-Fluor- oder 4-Chlorphenyl bedeutet, herstellt.

**9.** Verfahren gemäss Anspruch 1 oder 2, dadurch gekennzeichnet, dass man Verbindungen der Formel I und ihre Salze, worin $Ar_1$ 2- oder 4-Fluor-, 3-Trifluormethyl-, 4-Chlor-, 3- oder 4-Dimethylamino- oder 3- oder 4-Acetylamino-phenyl bedeutet und $Ar_2$ 4-Fluor- oder 4-Chlorphenyl bedeutet, herstellt.

**10.** Verfahren gemäss Anspruch 1 oder 2, dadurch gekennzeichnet, dass man Verbindungen der Formel I und ihre Salze, worin $Ar_1$ durch Methyl, Methoxy, Fluor, Chlor oder Trifluormethyl monosubstituiertes oder durch Methoxy disubstituiertes Phenyl bedeutet und $Ar_2$ durch Fluor oder Chlor monosubstituiertes Phenyl bedeutet, herstellt.

**11.** Verfahren gemäss Anspruch 1 oder 2, dadurch gekennzeichnet, dass man Verbindungen der Formel I und ihre Salze, worin $Ar_1$ durch Methyl oder Fluor monosubstituiertes Phenyl bedeutet und $Ar_2$ durch Fluor oder Chlor monosubstituiertes Phenyl bedeutet, herstellt.

**12.** Verfahren gemäss Anspruch 1 oder 2, dadurch gekennzeichnet, dass man Verbindungen der Formel I und ihre Salze, worin $Ar_1$ 2- oder 4-Fluorphenyl, 2-Methylphenyl oder 3,5-Dimethoxyphenyl bedeutet und $Ar_2$ 4-Fluor- oder 4-Chlorphenyl bedeutet, herstellt.

**13.** Verfahren gemäss Anspruch 1 oder 2, dadurch gekennzeichnet, dass man
1-[2-(4-Chlorphenyl)-ethyl]-4-(4-chlorbenzoyl)-piperazin,
1-[2-(4-Chlorphenyl)-ethyl]-4-(3-chlorbenzoyl)-piperazin,
1-[2-((4-Chlorphenyl)-ethyl]-4-(2-chlorbenzoyl)-piperazin,
1-[2-(4-Chlorphenyl)-ethyl]-4-(2,6-dichlorbenzoyl)-piperazin,
1-[2-(4-Chlorphenyl)-ethyl]-4-(2-fluorbenzoyl)-piperazin,
1-[2-(4-Chlorphenyl)-ethyl]-4-(2,4-dichlorbenzoyl)-piperazin,
1-[2-(4-Chlorphenyl)-ethyl]-4-(4-fluorbenzoyl)-piperazin,
1-[2-(4-Chlorphenyl)-ethyl]-4-(3,4-dichlorbenzoyl)-piperazin,
1-[2-(4-Chlorphenyl)-ethyl]-4-(3-fluorbenzoyl)-piperazin,
1-[2-(4-Chlorphenyl)-ethyl]-4-(2-aminobenzoyl)-piperazin,
1-(2-Phenylethyl)-4-(2-amino-benzoyl)-piperazin,
1-[2-(4-Chlorphenyl)-ethyl]-4-(3-methoxy-benzoyl)-piperazin,
1-[2-(4-Chlorphenyl)-ethyl]-4-(3,4-dimethoxybenzoyl)-piperazin,
1-[2-(4-Chlorphenyl)-ethyl]-4-(4-methoxybenzoyl)-piperazin,
1-[2-(4-Chlorphenyl)-ethyl]-4-(4-methylbenzoyl)-piperazin,
1-[2-(4-Chlorphenyl)-ethyl]-4-(2-methoxybenzoyl)-piperazin,
1-[2-(4-Chlorphenyl)-ethyl]-4-(5-cyano-2-methoxy-benzoyl)-piperazin,
1-[2-(4-Methoxyphenyl)-ethyl]-4-(3,4-dimethoxybenzoyl)-piperazin,
1-[2-(4-Methoxyphenyl)-ethyl]-4-(3-methoxybenzoyl)-piperazin,
1-[2-(3-Chlorphenyl)-ethyl]-4-(3,4-dimethoxybenzoyl)-piperazin,
1-[2-(3-Chlorphenyl)-ethyl]-4-(3-methoxybenzoyl)-piperazin,
1-(2-Phenylethyl)-4-(3,4-dimethoxybenzoyl)-piperazin,
1-(2-Phenylethyl)-4-(3-methoxybenzoyl)-piperazin,
1-[2-(4-Fluorphenyl)-ethyl]-4-(3,4-dimethoxybenzoyl)-piperazin,
1-[2-(4-Fluorphenyl)-ethyl]-4-(4-chlorbenzoyl)-piperazin,
1-[2-(4-Fluorphenyl)-ethyl]-4-(4-fluorbenzoyl)-piperazin,
1-[2-(4-Fluorphenyl)-ethyl-4-(3-methoxybenzoyl)-piperazin,
1-[2-(4-Chlorphenyl)-ethyl]-4-(2-trifluormethylbenzoyl)-piperazin,
1-[2-(4-Chlorphenyl)-ethyl]-4-(3-trifluormethylbenzoyl)-piperazin,
1-[2-(4-Chlorphenyl)-ethyl]-4-(4-trifluormethylbenzoyl)-piperazin,
1-[2-(4-Chlorphenyl)-ethyl]-4-(4-methylbenzoyl)-piperazin,
1-[2-(4-Chlorphenyl)-ethyl]-4-(2-methylbenzoyl)-piperazin,
1-[2-(4-Chlorphenyl)-ethyl]-4-(2,6-dimethylbenzoyl)-piperazin,
1-[2-(4-Chlorphenyl)-ethyl]-4-(3,5-dichlorbenzoyl)-piperazin,
1-[2-(4-Chlorphenyl)-ethyl]-4-(2,6-dimethoxybenzoyl)-piperazin

oder ein Salz davon herstellt.

**14.** Verfahren gemäss Anspruch 1, dadurch gekennzeichnet, dass man 1-[2-(4-Chlorphenyl)-ethyl]-4-(2-fluorobenzoyl)piperazin oder ein Salz davon herstellt.

**15.** Verfahren gemäss Anspruch 1, dadurch gekennzeichnet, dass man 1-[2-(4-Chlorphenyl)-ethyl]-4-(3-dimethylaminobenzoyl)piperazin oder ein Salz davon herstellt.

**16.** Verfahren gemäss Anspruch 1, dadurch gekennzeichnet, dass man 1-[2-(4-Chlorphenyl)-ethyl]-4-(4-dimethylaminobenzoyl)piperazin oder ein Salz davon herstellt.

**17.** Verfahren gemäss Anspruch 1, dadurch gekennzeichnet, dass man 1-[2-(4-Chlorphenyl)-ethyl]-4-(4-acetaminobenzoyl)piperazin oder ein Salz davon herstellt.

**18.** Verfahren gemäss Anspruch 1 oder 2, dadurch gekennzeichnet, dass man 1-[2-(4-Chlorphenyl)-ethyl]-4-(3,5-dimethoxybenzoyl)piperazin oder ein Salz davon herstellt.

**19.** Verfahren gemäss Anspruch 1, dadurch gekennzeichnet, dass man 1-[2-(p-Chlorphenyl)-ethyl]-4-(3-acetamidobenzoyl)piperazin oder ein Salz davon herstellt.

**20.** Verfahren zur Herstellung pharmazeutischer Präparate, dadurch gekennzeichnet, dass man eine Verbindung gemäss einem der Ansprüche 1-19 in freier Form oder in Form eines pharmazeutisch verwendbaren Salzes, gegebenenfalls unter Beimischung von üblichen Hilfs- und Trägerstoffen, zu pharmazeutischen Präparaten verarbeitet.

**21.** Verbindungen der Formel

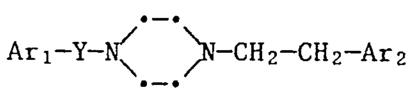

(V)

und ihre Salze, worin Y für $-CH_2-$ steht und $Ar_1$ und $Ar_2$ die gemäss einem der Ansprüche 1-12 angegebenen Bedeutungen haben.

**Claims**

**Claims for the following Contracting States : BE, CH, DE, FR, GB, IT, LI, LU, NL, SE**

**1.** A piperazine of formula

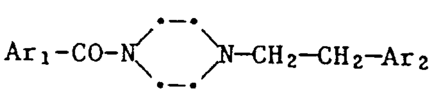

(I)

or a salt thereof, in which $Ar_1$ represents phenyl that is unsubstituted or mono- or di-substituted by $C_1$-$C_7$alkyl,$C_1$-$C_7$alkoxy, cyano, halogen, trifluoromethyl, amino, $C_1$-$C_7$alkylamino, di-$C_1$-$C_7$alkylamino and/or by $C_1$-$C_7$alkanoylamino, and in which $Ar_2$ represents phenyl that is unsubstituted or mono- or di-substituted by $C_1$-$C_7$alkyl,$C_1$-$C_7$alkoxy or by halogen.

**2.** A compound according to claim 1 of formula I or a salt thereof in which $Ar_1$ represents phenyl that is monosubstituted by halogen having an atomic number of up to and including 35, by trifluoromethyl, by di-$C_1$-$C_4$alkylaminoor by $C_2$-$C_5$alkanoylamino, or represents phenyl that is disubstituted by $C_1$-$C_4$alkyl or by $C_1$-$C_4$alkoxy, and $Ar_2$ represents phenyl that is monosubstituted by halogen having an atomic number of up to and including 35.

**3.** A compound according to claim 1 of formula I or a salt thereof in which $Ar_1$ represents phenyl that is unsubstituted or mono- or di-substituted by $C_1$-$C_4$alkyl, $C_1$-$C_4$alkoxy, halogen, trifluoromethyl, di-$C_1$-

$C_4$alkylamino and/or by $C_1$-$C_5$alkanoylamino, and $Ar_2$ represents phenyl that is unsubstituted or mono- or di-substituted by $C_1$-$C_4$alkoxy or by halogen.

**4.** A compound according to claim 1 of formula I or a salt thereof in which $Ar_1$ represents phenyl that is unsubstituted or mono- or di-substituted by $C_1$-$C_4$alkyl, $C_1$-$C_4$alkoxy, halogen and/or by trifluoromethyl, and $Ar_2$ represents phenyl that is monosubstituted by halogen.

**5.** A compound according to claim 1 of formula I or a salt thereof in which $Ar_1$ represents phenyl that is unsubstituted or mono- or di-substituted by $C_1$-$C_4$alkyl, $C_1$-$C_4$alkoxy, halogen and/or by trifluoromethyl, and $Ar_2$ represents phenyl that is monosubstituted by $C_1$-$C_4$alkoxy.

**6.** A compound according to claim 1 of formula I or a salt thereof in which $Ar_1$ represents 3,5-di-$C_1$-$C_4$alkoxyphenyl and $Ar_2$ represents 4-halophenyl.

**7.** A compound according to claim 1 of formula I or a salt thereof in which $Ar_1$ represents 2- or 4-fluorophenyl, 2- or 4-chlorophenyl, 2- or 3-trifluoromethylphenyl, 3- or 4-dimethylaminophenyl, 3- or 4-acetylaminophenyl, 3,4- or 3,5-dimethoxyphenyl or 2,6-dimethylphenyl, and $Ar_2$ represents 4-fluoro- or 4-chloro-phenyl.

**8.** A compound according to claim 1 of formula I or a salt thereof in which $Ar_1$ represents 2- or 4-fluorophenyl, 3-trifluoromethylphenyl, 4-chlorophenyl, 3- or 4-dimethylaminophenyl or 3- or 4-acetylaminophenyl, and $Ar_2$ represents 4-fluoro- or 4-chloro-phenyl.

**9.** A compound according to claim 1 of formula I or a salt thereof in which $Ar_1$ represents phenyl that is monosubstituted by methyl, methoxy, fluorine, chlorine or by trifluoromethyl or that is disubstituted by methoxy, and $Ar_2$ represents phenyl that is monosubstituted by fluorine or chlorine.

**10.** A compound according to claim 1 of formula I or a salt thereof in which $Ar_1$ represents phenyl that is monosubstituted by methyl or fluorine, and $Ar_2$ represents phenyl that is monosubstituted by fluorine or chlorine.

**11.** A compound according to claim 1 of formula I or a salt thereof in which $Ar_1$ represents 2- or 4-fluorophenyl, 2-methylphenyl or 3,5-dimethoxyphenyl, and $Ar_2$ represents 4-fluoro- or 4-chloro-phenyl.

**12.** 1-[2-(4-chlorophenyl)-ethyl]-4-(4-chlorobenzoyl)-piperazine,
1-[2-(4-chlorophenyl)-ethyl]-4-(3-chlorobenzoyl)-piperazine,
1-[2-(4-chlorophenyl)-ethyl]-4-(2-chlorobenzoyl)-piperazine,
1-[2-(4-chlorophenyl)-ethyl]-4-(2,6-dichlorobenzoyl)-piperazine,
1-[2-(4-chlorophenyl)-ethyl]-4-(2-fluorobenzoyl)-piperazine,
1-[2-(4-chlorophenyl)-ethyl]-4-(2,4-dichlorobenzoyl)-piperazine,
1-[2-(4-chlorophenyl)-ethyl]-4-(4-fluorobenzoyl)-piperazine,
1-[2-(4-chlorophenyl)-ethyl]-4-(3,4-dichlorobenzoyl)-piperazine,
1-[2-(4-chlorophenyl)-ethyl]-4-(3-fluorobenzoyl)-piperazine,
1-[2-(4-chlorophenyl)-ethyl]-4-(2-aminobenzoyl)-piperazine,
1-(2-phenylethyl)-4-(2-aminobenzoyl)-piperazine,
1-[2-(4-chlorophenyl)-ethyl]-4-(3-methoxybenzoyl)-piperazine,
1-[2-(4-chlorophenyl)-ethyl]-4-(3,4-dimethoxybenzoyl)-piperazine,
1-[2-(4-chlorophenyl)-ethyl]-4-(4-methoxybenzoyl)-piperazine,
1-[2-(4-chlorophenyl)-ethyl]-4-(4-methylbenzoyl)-piperazine,
1-[2-(4-chlorophenyl)-ethyl]-4-(2-methoxybenzoyl)-piperazine,
1-[2-(4-chlorophenyl)-ethyl]-4-(5-cyano-2-methoxybenzoyl)-piperazine,
1-[2-(4-methoxyphenyl)-ethyl]-4-(3,4-dimethoxybenzoyl)-piperazine,
1-[2-(4-methoxyphenyl)-ethyl]-4-(3-methoxybenzoyl)-piperazine,
1-[2-(3-chlorophenyl)-ethyl]-4-(3,4-dimethoxybenzoyl)-piperazine,
1-[2-(3-chlorophenyl)-ethyl]-4-(3-methoxybenzoyl)-piperazine,
1-(2-phenylethyl)-4-(3,4-dimethoxybenzoyl)-piperazine,
1-(2-phenylethyl)-4-(3-methoxybenzoyl)-piperazine,
1-[2-(4-fluorophenyl)-ethyl]-4-(3,4-dimethoxybenzoyl)-piperazine,

1-[2-(4-fluorophenyl)-ethyl]-4-(4-chlorobenzoyl)-piperazine,
1-[2-(4-fluorophenyl)-ethyl]-4-(4-fluorobenzoyl)-piperazine,
1-[2-(4-fluorophenyl)-ethyl]-4-(3-methoxybenzoyl)-piperazine,
1-[2-(4-chlorophenyl)-ethyl]-4-(2-trifluoromethylbenzoyl)-piperazine,
1-[2-(4-chlorophenyl)-ethyl]-4-(3-trifluoromethylbenzoyl)-piperazine,
1-[2-(4-chlorophenyl)-ethyl]-4-(4-trifluoromethylbenzoyl)-piperazine,
1-[2-(4-chlorophenyl)-ethyl]-4-(4-methylbenzoyl)-piperazine,
1-[2-(4-chlorophenyl)-ethyl]-4-(2-methylbenzoyl)-piperazine,
1-[2-(4-chlorophenyl)-ethyl]-4-(2,6-dimethylbenzoyl)-piperazine,
1-[2-(4-chlorophenyl)-ethyl]-4-(3,5-dichlorobenzoyl)-piperazine,
1-[2-(4-chlorophenyl)-ethyl]-4-(2,6-dimethoxybenzoyl)-piperazine,
or a salt thereof.

**13.** 1-[2-(4-chlorophenyl)-ethyl]-4-(2-fluorobenzoyl)-piperazine or a salt thereof.

**14.** 1-[2-(4-chlorophenyl)-ethyl]-4-(3-dimethylaminobenzoyl)-piperazine or a salt thereof.

**15.** 1-[2-(4-chlorophenyl)-ethyl]-4-(4-dimethylaminobenzoyl)-piperazine or a salt thereof.

**16.** 1-[2-(4-chlorophenyl)-ethyl]-4-(4-acetaminobenzoyl)-piperazine or a salt thereof.

**17.** 1-[2-(4-chlorophenyl)-ethyl]-4-(3,5-dimethoxybenzoyl)-piperazine or a salt thereof.

**18.** 1-[2-(p-chlorophenyl)-ethyl]-4-(3-acetamidobenzoyl)-piperazine or a salt thereof.

**19.** A compound according to any one of claims 1 to 18 for use in a method for the therapeutic or prophylactic treatment of the human or animal body.

**20.** A compound according to any one of claims 1 to 18 for use as an analgesic.

**21.** A pharmaceutical composition comprising a compound according to any one of claims 1 to 18 in free form or in the form of a pharmaceutically acceptable salt, together with a pharmaceutically acceptable carrier.

**22.** The use of a compound according to any one of claims 1 to 18 in the preparation of an analgesic.

**23.** A process for the preparation of a compound according to any one of claims 1 to 18, which process comprises

a) reacting a compound of formula

$Ar_1$-$X_1$ (IIa),

or a salt thereof, in which $X_1$ represents carboxy or reactive functionally modified carboxy, with a compound of formula

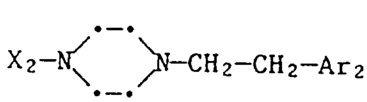

(IIb),

or a salt thereof, in which $X_2$ represents hydrogen or an amino-protecting group, or

b) reacting a compound of formula

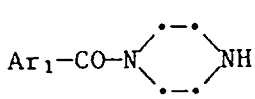

(IIIa),

or a salt thereof, with a compound of formula

$X_3$-$CH_2$-$CH_2$-$Ar_2$ (IIIb),

or a salt thereof, in which $X_3$ represents hydroxy or reactive esterified hydroxy, or
c) intramolecularly condensing a compound of formula

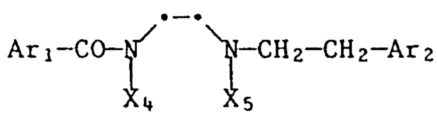

(IV),

or a salt thereof, in which one of the radicals $X_4$ and $X_5$ is hydrogen and the other is a group of the formula -$CH_2$-$CH_2$-$X_3$ and $X_3$ represents hydroxy or reactive esterified hydroxy, or
d) oxidising a compound of formula

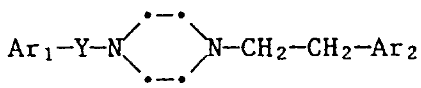

(V),

or a salt thereof, in which Y represents a group that can be oxidised to -CO-,
and, if desired, converting a compound obtainable in accordance with the process or by other means into a different compound of formula I, separating an isomeric mixture obtainable in accordance with the process into its components, converting a free compound of formula I obtainable in accordance with the process into a salt and/or converting a salt obtainable in accordance with the process into the free compound of formula I or into a different salt.

**24.** A process for the preparation of a compound of formula I according to any one of claims 1 to 18, or a salt thereof, in which $Ar_1$ represents phenyl that is mono- or di-substituted by $C_1$-$C_7$alkoxy, which process comprises alkylating a compound of formula

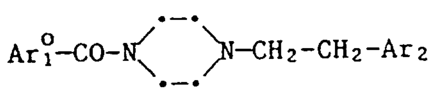

(VI),

or a salt thereof, in which $Ar_1^o$ represents phenyl that is mono- or di-substituted by hydroxy,
and, if desired, converting a compound obtainable in accordance with the process or by other means into a different compound of formula I, separating an isomeric mixture obtainable in accordance with the process into its components, converting a free compound of formula I obtainable in accordance with the process into a salt and/or converting a salt obtainable in accordance with the process into the free compound of formula I or into a different salt.

**25.** A process for the preparation of a pharmaceutical composition according to claim 21, which process comprises processing a compound according to any one of claims 1 to 18 in free form or in the form of a pharmaceutically acceptable salt, optionally with the admixture of customary excipients and carriers, to form a pharmaceutical composition.

**26.** A compound of formula

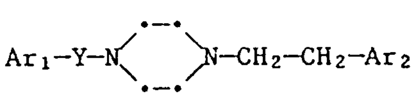

(V),

or a salt thereof, in which Y is -$CH_2$- and $Ar_1$ and $Ar_2$ are as defined in any one of claims 1 to 11.

**27.** The use of a compound according to claim 26 in the preparation of an analgesic.

**Claims for the following Contracting States : AT, ES, GR**

**1.** A process for the preparation of a piperazine of formula

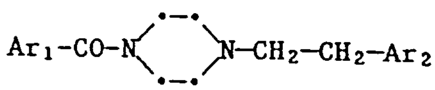

(I)

or a salt thereof, in which $Ar_1$ represents phenyl that is unsubstituted or mono- or di-substituted by $C_1$-$C_7$alkyl,$C_1$-$C_7$alkoxy, cyano, halogen, trifluoromethyl, amino, $C_1$-$C_7$alkylamino, di-$C_1$-$C_7$alkylamino and/or by $C_1$-$C_7$alkanoylamino, and in which $Ar_2$ represents phenyl that is unsubstituted or mono- or di-substituted by $C_1$-$C_7$alkyl,$C_1$-$C_7$alkoxy or by halogen,
which process comprises

a) reacting a compound of formula

$Ar_1$-$X_1$ (IIa),

or a salt thereof, in which $X_1$ represents carboxy or reactive functionally modified carboxy, with a compound of formula

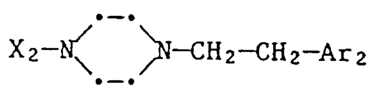

(IIb),

or a salt thereof, in which $X_2$ represents hydrogen or an amino-protecting group, or

b) reacting a compound of formula

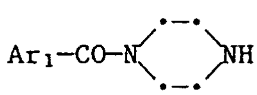

(IIIa),

or a salt thereof, with a compound of formula

$X_3$-$CH_2$-$CH_2$-$Ar_2$ (IIIb),

or a salt thereof, in which $X_3$ represents hydroxy or reactive esterified hydroxy, or

c) intramolecularly condensing a compound of formula

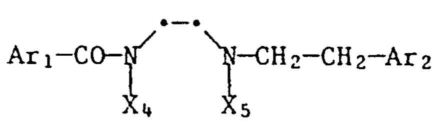

(IV),

or a salt thereof, in which one of the radicals $X_4$ and $X_5$ is hydrogen and the other is a group of the formula -$CH_2$-$CH_2$-$X_3$ and $X_3$ represents hydroxy or reactive esterified hydroxy, or

d) oxidising a compound of formula

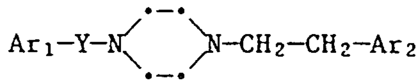

(V),

or a salt thereof, in which Y represents a group that can be oxidised to -CO-,

and, if desired, converting a compound obtainable in accordance with the process or by other means into a different compound of formula I, separating an isomeric mixture obtainable in accordance with the process into its components, converting a free compound of formula I obtainable in accordance with the process into a salt and/or converting a salt obtainable in accordance with the process into the free compound of formula I or into a different salt.

**2.** A process for the preparation of a compound of formula I shown in claim 1, or a salt thereof, in which $Ar_1$ represents phenyl that is mono- or di-substituted by $C_1$-$C_7$alkoxy, which process comprises alkylating a compound of formula

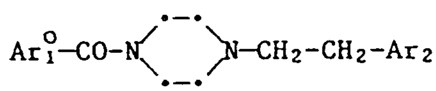

(VI),

or a salt thereof, in which $Ar_1^o$ represents phenyl that is mono- or di-substituted by hydroxy,

and, if desired, converting a compound obtainable in accordance with the process or by other means into a different compound of formula I, separating an isomeric mixture obtainable in accordance with the process into its components, converting a free compound of formula I obtainable in accordance with the process into a salt and/or converting a salt obtainable in accordance with the process into the free compound of formula I or into a different salt.

**3.** A process according to either claim 1 or claim 2 wherein there is prepared a compound of formula I or a salt thereof in which $Ar_1$ represents phenyl that is monosubstituted by halogen having an atomic number of up to and including 35, by trifluoromethyl, by di-$C_1$-$C_4$alkylamino or by $C_2$-$C_5$alkanoylamino, or represents phenyl that is disubstituted by $C_1$-$C_4$alkyl or by $C_1$-$C_4$alkoxy, and $Ar_2$ represents phenyl that is monosubstituted by halogen having an atomic number of up to and including 35.

**4.** A process according to either claim 1 or claim 2 wherein there is prepared a compound of formula I or a salt thereof in which $Ar_1$ represents phenyl that is unsubstituted or mono- or di-substituted by $C_1$-$C_4$alkyl,$C_1$-$C_4$alkoxy, halogen, trifluoromethyl, di-$C_1$-$C_4$alkylamino and/or by $C_1$-$C_5$alkanoylamino, and $Ar_2$ represents phenyl that is unsubstituted or mono- or di-substituted by $C_1$-$C_4$alkoxy or by halogen.

**5.** A process according to either claim 1 or claim 2 wherein there is prepared a compound of formula I or a salt thereof in which $Ar_1$ represents phenyl that is unsubstituted or mono- or di-substituted by $C_1$-$C_4$alkyl,$C_1$-$C_4$alkoxy, halogen and/or by trifluoromethyl, and $Ar_2$ represents phenyl that is monosubstituted by halogen.

**6.** A process according to either claim 1 or claim 2 wherein there is prepared a compound of formula I or a salt thereof in which $Ar_1$ represents phenyl that is unsubstituted or mono- or di-substituted by $C_1$-$C_4$alkyl,$C_1$-$C_4$alkoxy, halogen and/or by trifluoromethyl, and $Ar_2$ represents phenyl that is monosubstituted by $C_1$-$C_4$alkoxy.

**7.** A process according to either claim 1 or claim 2 wherein there is prepared a compound of formula I or a salt thereof in which $Ar_1$ represents 3,5-di-$C_1$-$C_4$alkoxyphenyl and $Ar_2$ represents 4-halophenyl.

**8.** A process according to either claim 1 or claim 2 wherein there is prepared a compound of formula I or a salt thereof in which $Ar_1$ represents 2- or 4-fluorophenyl, 2- or 4-chlorophenyl, 2- or 3-trifluoromethylphenyl, 3- or 4-dimethylaminophenyl, 3- or 4-acetylaminophenyl, 3,4- or 3,5-dimethoxyphenyl or 2,6-

dimethylphenyl, and $Ar_2$ represents 4-fluoro- or 4-chloro-phenyl.

**9.** A process according to either claim 1 or claim 2 wherein there is prepared a compound of formula I or a salt thereof in which $Ar_1$ represents 2- or 4-fluorophenyl, 3-trifluoromethylphenyl, 4-chlorophenyl, 3- or 4-dimethylaminophenyl or 3- or 4-acetylaminophenyl, and $Ar_2$ represents 4-fluoro- or 4-chloro-phenyl.

**10.** A process according to either claim 1 or claim 2 wherein there is prepared a compound of formula I or a salt thereof in which $Ar_1$ represents phenyl that is monosubstituted by methyl, methoxy, fluorine, chlorine or by trifluoromethyl or that is disubstituted by methoxy, and $Ar_2$ represents phenyl that is monosubstituted by fluorine or chlorine.

**11.** A process according to either claim 1 or claim 2 wherein there is prepared a compound of formula I or a salt thereof in which $Ar_1$ represents phenyl that is monosubstituted by methyl or fluorine, and $Ar_2$ represents phenyl that is monosubstituted by fluorine or chlorine.

**12.** A process according to either claim 1 or claim 2 wherein there is prepared a compound of formula I or a salt thereof in which $Ar_1$ represents 2- or 4-fluorophenyl, 2-methylphenyl or 3,5-dimethoxyphenyl, and $Ar_2$ represents 4-fluoro- or 4-chloro-phenyl.

**13.** A process according to either claim 1 or claim 2 wherein there is prepared
1-[2-(4-chlorophenyl)-ethyl]-4-(4-chlorobenzoyl)-piperazine,
1-[2-(4-chlorophenyl)-ethyl]-4-(3-chlorobenzoyl)-piperazine,
1-[2-(4-chlorophenyl)-ethyl]-4-(2-chlorobenzoyl)-piperazine,
1-[2-(4-chlorophenyl)-ethyl]-4-(2,6-dichlorobenzoyl)-piperazine,
1-[2-(4-chlorophenyl)-ethyl]-4-(2-fluorobenzoyl)-piperazine,
1-[2-(4-chlorophenyl)-ethyl]-4-(2,4-dichlorobenzoyl)-piperazine,
1-[2-(4-chlorophenyl)-ethyl]-4-(4-fluorobenzoyl)-piperazine,
1-[2-(4-chlorophenyl)-ethyl]-4-(3,4-dichlorobenzoyl)-piperazine,
1-[2-(4-chlorophenyl)-ethyl]-4-(3-fluorobenzoyl)-piperazine,
1-[2-(4-chlorophenyl)-ethyl]-4-(2-aminobenzoyl)-piperazine,
1-(2-phenylethyl)-4-(2-aminobenzoyl)-piperazine,
1-[2-(4-chlorophenyl)-ethyl]-4-(3-methoxybenzoyl)-piperazine,
1-[2-(4-chlorophenyl)-ethyl]-4-(3,4-dimethoxybenzoyl)-piperazine,
1-[2-(4-chlorophenyl)-ethyl]-4-(4-methoxybenzoyl)-piperazine,
1-[2-(4-chlorophenyl)-ethyl]-4-(4-methylbenzoyl)-piperazine,
1-[2-(4-chlorophenyl)-ethyl]-4-(2-methoxybenzoyl)-piperazine,
1-[2-(4-chlorophenyl)-ethyl]-4-(5-cyano-2-methoxybenzoyl)-piperazine,
1-[2-(4-methoxyphenyl)-ethyl]-4-(3,4-dimethoxybenzoyl)-piperazine,
1-[2-(4-methoxyphenyl)-ethyl]-4-(3-methoxybenzoyl)-piperazine,
1-[2-(3-chlorophenyl)-ethyl]-4-(3,4-dimethoxybenzoyl)-piperazine,
1-[2-(3-chlorophenyl)-ethyl]-4-(3-methoxybenzoyl)-piperazine,
1-(2-phenylethyl)-4-(3,4-dimethoxybenzoyl)-piperazine,
1-(2-phenylethyl)-4-(3-methoxybenzoyl)-piperazine,
1-[2-(4-fluorophenyl)-ethyl]-4-(3,4-dimethoxybenzoyl)-piperazine,
1-[2-(4-fluorophenyl)-ethyl]-4-(4-chlorobenzoyl)-piperazine,
1-[2-(4-fluorophenyl)-ethyl]-4-(4-fluorobenzoyl)-piperazine,
1-[2-(4-fluorophenyl)-ethyl]-4-(3-methoxybenzoyl)-piperazine,
1-[2-(4-chlorophenyl)-ethyl]-4-(2-trifluoromethylbenzoyl)-piperazine,
1-[2-(4-chlorophenyl)-ethyl]-4-(3-trifluoromethylbenzoyl)-piperazine,
1-[2-(4-chlorophenyl)-ethyl]-4-(4-trifluoromethylbenzoyl)-piperazine,
1-[2-(4-chlorophenyl)-ethyl]-4-(4-methylbenzoyl)-piperazine,
1-[2-(4-chlorophenyl)-ethyl]-4-(2-methylbenzoyl)-piperazine,
1-[2-(4-chlorophenyl)-ethyl]-4-(2,6-dimethylbenzoyl)-piperazine,
1-[2-(4-chlorophenyl)-ethyl]-4-(3,5-dichlorobenzoyl)-piperazine,
1-[2-(4-chlorophenyl)-ethyl]-4-(2,6-dimethoxybenzoyl)-piperazine,
or a salt thereof.

**14.** A process according to claim 1 wherein there is prepared 1-[2-(4-chlorophenyl)-ethyl]-4-(2-fluorobenzoyl)-piperazine or a salt thereof.

**15.** A process according to claim 1 wherein there is prepared 1-[2-(4-chlorophenyl)-ethyl]-4-(3-dimethylaminobenzoyl)-piperazine or a salt thereof.

**16.** A process according to claim 1 wherein there is prepared 1-[2-(4-chlorophenyl)-ethyl]-4-(4-dimethylaminobenzoyl)-piperazine or a salt thereof.

**17.** A process according to claim 1 wherein there is prepared 1-[2-(4-chlorophenyl)-ethyl]-4-(4-acetaminobenzoyl)-piperazine or a salt thereof.

**18.** A process according to either claim 1 or claim 2 wherein there is prepared 1-[2-(4-chlorophenyl)-ethyl]-4-(3,5-dimethoxybenzoyl)-piperazine or a salt thereof.

**19.** A process according to claim 1 wherein there is prepared 1-[2-(p-chlorophenyl)-ethyl]-4-(3-acetamidobenzoyl)-piperazine or a salt thereof.

**20.** A process for the preparation of a pharmaceutical composition, which process comprises processing a compound according to any one of claims 1 to 19 in free form or in the form of a pharmaceutically acceptable salt, optionally with the admixture of customary excipients and carriers, to form a pharmaceutical composition.

**21.** A compound of formula

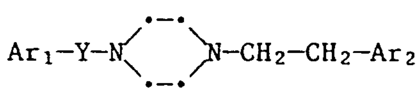

(V),

or a salt thereof, in which Y is $-CH_2-$ and $Ar_1$ and $Ar_2$ are as defined in any one of claims 1 to 12.

**Revendications**
**Revendications pour les Etats contractants suivants : BE, CH, DE, FR, GB, IT, LI, LU, NL, SE**

**1.** Pipérazines de formule

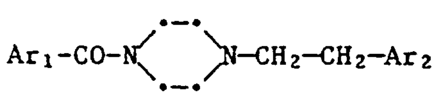

(I)

dans laquelle $Ar_1$ représente un groupe phényle non substitué ou mono- ou di-substitué par des groupes alkyle en C1-C7, alcoxy en C1-C7, cyano, des halogènes, des groupes trifluorométhyle, amino, alkylamino en C1-C7, di-(alkyle en C1-C7)-amino et/ou alcanoylamino en C1-C7 et
$Ar_2$ représente un groupe phényle non substitué ou mono- ou di-substitué par des groupes alkyle en C1-C7, alcoxy en C1-C7 ou des halogènes,
et leurs sels.

**2.** Composés selon revendication 1, de formule I dans laquelle $Ar_1$ représente un groupe phényle monosubstitué par un halogène de numéro atomique allant jusqu'à 35 inclus, par un groupe trifluorométhyle, par un groupe di-(alkyle en C1-C3)-amino ou par un groupe alcanoylamino en C2-C5, ou un groupe phényle disubstitué par des groupes alkyle en C1-C4 ou par des groupes alcoxy en C1-C4, et
$Ar_2$ représente un groupe phényle monosubstitué par un halogène de numéro atomique allant jusqu'à 35 inclus,
et leurs sels.

3. Composés selon revendication 1, de formule I dans laquelle $Ar_1$ représente un groupe phényle non substitué ou mono- ou di-substitué par des groupes alkyle en C1-C4, alcoxy en C1-C4, des halogènes, des groupes trifluorométhyle, di-(alkyle en C1-C4)-amino et/ou alcanoylamino en C1-C5 et $Ar_2$ représente un groupe phényle non substitué ou mono- ou disubstitué par des groupes alcoxy en C1-C4 ou des halogènes,
et leurs sels.

4. Composés selon revendication 1, de formule I dans laquelle $Ar_1$ représente un groupe phényle non substitué ou mono- ou di-substitué par des groupes alkyle en C1-C4, alcoxy en C1-C4, des halogènes et/ou des groupes trifluorométhyle, et $Ar_2$ représente un groupe phényle monosubstitué par un halogène,
et leurs sels.

5. Composés selon revendication 1, de formule I dans laquelle $Ar_1$ représente un groupe phényle non substitué ou mono- ou di-substitué par des groupes alkyle en C1-C4, alcoxy en C1-C4, des halogènes et/ou des groupes trifluorométhyle, et $Ar_2$ représente un groupe phényle monosubstitué par un groupe alcoxy en C1-C4,
et leurs sels.

6. Composés selon revendication 1, de formule I dans laquelle $Ar_1$ représente un groupe 3,5-di-(alcoxy en C1-C4)-phényle et $Ar_2$ un groupe 4-halogénophényle,
et leurs sels.

7. Composés selon revendication 1, de formule I, dans laquelle $Ar_1$ représente un groupe 2- ou 4-fluoro-, 2- ou 4-chloro-, 2- ou 3-trifluorométhyl-, 3- ou 4-diméthylamino-, 3- ou 4-acétylamino-, 3,4- ou 3,5-diméthoxy- ou 2,6-diméthylphényle et $Ar_2$ un groupe 4-fluoro- ou 4-chloro-phényle,
et leurs sels.

8. Composés selon revendication 1, de formule I dans laquelle $Ar_1$ représente un groupe 2- ou 4-fluoro-, 3-trifluorométhyl-, 4-chloro-, 3- ou 4-diméthylamino- ou 3- ou 4-acétylamino-phényle et $Ar_2$ un groupe 4-fluoro- ou 4-chloro-phényle,
et leurs sels.

9. Composés selon revendication 1, de formule I dans laquelle $Ar_1$ représente un groupe phényle monosubstitué par un groupe méthyle, méthoxy, le fluor, le chlore ou un groupe trifluorométhyle, ou disubstitué par des groupe méthoxy, et $Ar_2$ représente un groupe phényle monosubstitué par le fluor ou le chlore,
et leurs sels.

10. Composés selon revendication 1, de formule I dans laquelle $Ar_1$ représente un groupe phényle monosubstitué par un groupe méthyle ou le fluor et $Ar_2$ un groupe phényle monosubstitué par le fluor ou le chlore,
et leurs sels.

11. Composés selon revendication 1, de formule I dans laquelle $Ar_1$ représente un groupe 2- ou 4-fluorophényle, 2-méthylphényle ou 3,5-diméthoxyphényle et $Ar_2$ un groupe 4-fluoro- ou 4-chlorophényle,
et leurs sels.

12. La 1-[2-(4-chlorophényl)-éthyl]-4-(4-chlorobenzoyl)-pipérazine,
la 1-[2-(4-chlorophényl)-éthyl]-4-(3-chlorobenzoyl)-pipérazine,
la 1-[2-((4-chlorophényl)-éthyl]-4-(2-chlorobenzoyl)-pipérazine,
la 1-[2-(4-chlorophényl)-éthyl]-4-(2,6-dichlorobenzoyl)-pipérazine,
la 1-[2-(4-chlorophényl)-éthyl]-4-(2-fluorobenzoyl)-pipérazine,
la 1-[2-(4-chlorophényl)-éthyl]-4-(2,4-dichlorobenzoyl)-pipérazine,
la 1-[2-(4-chlorophényl)-éthyl]-4-(4-fluorobenzoyl)-pipérazine,
la 1-[2-(4-chlorophényl)-éthyl]-4-[3,4-dichlorobenzoyl)-pipérazine,
la 1-[2-(4-chlorophényl)-éthyl]-4-(3-fluorobenzoyl)-pipérazine,

la 1-[2-(4-chlorophényl)-éthyl]-4-(2-aminobenzoyl)-pipérazine,
la 1-(2-phényléthyl)-4-(2-amino-benzoyl)-pipérazine,
la 1-[2-(4-chlorophényl)-éthyl]-4-(3-méthoxy-benzoyl)-pipérazine,
la 1-[2-(4-chlorophényl)-éthyl]-4-(3,4-diméthoxybenzoyl)-pipérazine,
la 1-[2-(4-chlorophényl)-éthyl]-4-(4-méthoxybenzoyl)-pipérazine,
la 1-[2-(4-chlorophényl)-éthyl]-4-(4-méthylbenzoyl)-pipérazine,
la 1-[2-(4-chlorophényl)-éthyl]-4-(2-méthoxybenzoyl)-pipérazine,
la 1-[2-(4-chlorophényl)-éthyl]-4-(5-cyano-2-méthoxybenzoyl)-pipérazine,
la 1-[2-(4-méthoxyphényl)-éthyl]-4-(3,4-diméthoxybenzoyl)-pipérazine,
la 1-[2-(4-méthoxyphényl)-éthyl]-4-(3-méthoxybenzoyl)-pipérazine,
la 1-[2-(3-chlorophényl)-éthyl]-4-(3,4-diméthoxybenzoyl)-pipérazine,
la 1-[2-(3-chlorophényl)-éthyl]-4-(3-méthoxybenzoyl)-pipérazine,
la 1-(2-phényléthyl)-4-(3,4-diméthoxybenzoyl)-pipérazine,
la 1-(2-phényléthyl)-4-(3-méthoxybenzoyl)-pipérazine,
la 1-[2-(4-fluorophényl)-éthyl]-4-(3,4-diméthoxybenzoyl)-pipérazine,
la 1-[2-(4-fluorophényl)-éthyl]-4-(4-chlorobenzoyl)-pipérazine,
la 1-[2-(4-fluorophényl)-éthyl]-4-(4-fluorobenzoyl)-pipérazine,
la 1-[2-(4-fluorophényl)-éthyl-4-(3-méthoxybenzoyl)-pipérazine,
la 1-[2-(4-chlorophényl)-éthyl]-4-(3-trifluorométhylbenzoyl)-pipérazine,
la 1-[2-(4-chlorophényl)-éthyl]-4-(3-trifluorométhylbenzoyl)-pipérazine,
la 1-[2-(4-chlorophényl)-éthyl]-4-(4-trifluorométhylbenzoyl)-pipérazine,
la 1-[2-(4-chlorophényl)-éthyl]-4-(4-méthylbenzoyl)-pipérazine,
la 1-[2-(4-chlorophényl)-éthyl]-4-(2-méthylbenzoyl)-pipérazine,
la 1-[2-(4-chlorophényl)-éthyl]-4-(2,6-diméthylbenzoyl)-pipérazine,
la 1-[2-(4-chlorophényl)-éthyl]-4-(3,5-dichlorobenzoyl)-pipérazine,
la 1-[2-(4-chlorophényl)-éthyl]-4-(2,6-diméthoxybenzoyl)-pipérazine
ou un de leurs sels.

**13.** La 1-[2-(4-chlorophényl)-éthyl]-4-(2-fluorobenzoyl)pipérazine ou l'un de ses sels.

**14.** La 1-[2-(4-chlorophényl)-éthyl]-4-(3-diméthylaminobenzoyl)pipérazine ou l'un de ses sels.

**15.** La 1-[2-(4-chlorophényl)-éthyl]-4-(4-diméthylaminobenzoyl)pipérazine ou l'un de ses sels.

**16.** La 1-[2-(4-chlorophényl)-éthyl]-4-(4-acétaminobenzoyl)pipérazine ou l'un de ses sels.

**17.** La 1-[2-(4-chlorophényl)-éthyl]-4-(3,5-diméthoxybenzoyl)pipérazine ou l'un de ses sels.

**18.** La 1-[2-(p-chlorophényl)-éthyl]-4-(3-acétamidobenzoyl)pipérazine ou l'un de ses sels.

**19.** Composé selon l'une des revendications 1 à 18, pour l'utilisation dans un procédé pour le traitement thérapeutique ou prophylactique de l'organisme humain ou animal.

**20.** Composé selon l'une des revendications 1 à 18, pour l'utilisation en tant qu'analgésique.

**21.** Compositions pharmaceutiques contenant un composé selon une des revendications 1 à 18 à l'état libre ou à l'état de sel acceptable pour l'usage pharmaceutique avec un véhicule acceptable pour l'usage pharmaceutique.

**22.** Utilisation de composés selon une des revendications 1 à 18 pour la préparation d'analgésiques.

**23.** Procédé de préparation des composés selon une des revendications 1 à 18, caractérisé en ce que
a) on fait réagir un composé de formule

$Ar_1$-$X_1$ (IIa)

ou l'un de ses sels, $X_1$ représentant un groupe carboxy ou un groupe carboxy qui a subi une modification fonctionnelle et qui est réactif, avec un composé de formule

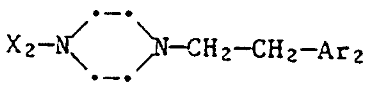

(IIb)

ou l'un de ses sels, $X_2$ représentant l'hydrogène ou un groupe protecteur du groupe amino, ou bien
b) on fait réagir un composé de formule

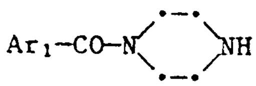

(IIIa)

ou l'un de ses sels, avec un composé de formule

$X_3$-$CH_2$-$CH_2$-$Ar_2$ (IIIb)

ou l'un de ses sels, $X_3$ représentant un groupe hydroxy ou un groupe hydroxy estérifié réactif, ou bien
c) on soumet un composé de formule

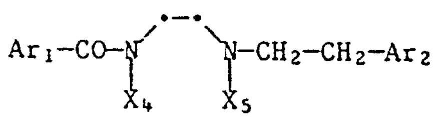

(IV)

ou l'un de ses sels, l'un des symboles $X_4$ et $X_5$ représentant l'hydrogène et l'autre un groupe de formule -$CH_2$-$CH_2$-$X_3$ dans lequel $X_3$ représente un groupe hydroxy ou un groupe hydroxy estérifié réactif, à une condensation intramoléculaire, ou bien
d) on oxyde un composé de formule

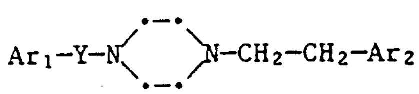

(V)

ou l'un de ses sels, Y représentant un groupe oxydable en groupe -CO-,
et si on le désire, on convertit un composé obtenu comme décrit ci-dessus ou de toute autre manière en un autre composé de formule I, on sépare un mélange d'isomères obtenu comme décrit ci-dessus en les composants, on convertit un composé de formule I à l'état libre obtenu comme décrit ci-dessus en un sel et/ou un sel obtenu comme décrit ci-dessus en le composé libre de formule I ou en un autre sel.

**24.** Procédé de préparation des composés de formule I selon une des revendications 1 à 18 et de leurs sels, pour lesquels $Ar_1$ représente un groupe phényle mono- ou di-substitué par des groupes alcoxy en C1-C7, caractérisé en ce que l'on alkyle un composé de formule

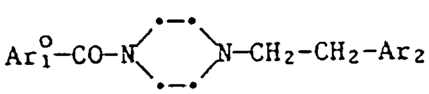

(VI)

ou l'un de ses sels, $Ar_1^o$ représentant un groupe phényle mono- ou di-substitué par des groupes hydroxy,
et si on le désire, on convertit un composé obtenu comme décrit ci-dessus ou de toute autre manière en un autre composé de formule I, on sépare un mélange d'isomères obtenu comme décrit ci-dessus en les composants, on convertit un composé de formule I obtenu à l'état libre comme décrit ci-dessus

en un sel et/ou un sel obtenu comme décrit ci-dessus en le composé libre de formule I ou en un autre sel.

**25.** Procédé de préparation de compositions pharmaceutiques selon revendication 21, caractérisé en ce que l'on met un composé selon une des revendications 1 à 18, à l'état libre ou à l'état de sel acceptable pour l'usage pharmaceutique, sous forme d'une composition pharmaceutique, éventuellement par mélange avec des produits auxiliaires et véhicules usuels.

**26.** Composés de formule

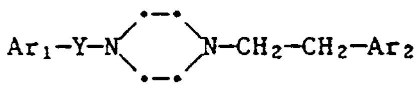

(V)

dans laquelle Y représente $-CH_2-$ et $Ar_1$ et $Ar_2$ ont les significations indiquées dans les revendications 1 à 11,
et leurs sels.

**27.** Utilisation des composés selon revendication 26 pour la préparation d'analgésiques.

**Revendications pour les Etats contractants suivants : AT, ES, GR**

**1.** Procédé de préparation des pipérazines de formule

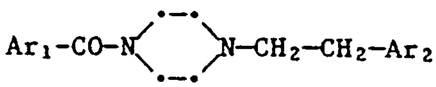

(I)

dans laquelle $Ar_1$ représente un groupe phényle non substitué ou mono- ou di-substitué par des groupes alkyle en C1-C7, alcoxy en C1-C7, cyano, des halogènes, des groupes trifluorométhyle, amino, alkylamino en C1-C7, di-(alkyle en C1-C7)-amino et/ou alcanoylamino en C1-C7 et $Ar_2$ représente un groupe phényle non substitué ou mono- ou di-substitué par des groupes alkyle en C1-C7, alcoxy en C1-C7 ou des halogènes,
et de leurs sels, caractérisé en ce que

a) on fait réagir un composé de formule

$Ar_1$-$X_1$ (IIa)

ou l'un de ses sels, $X_1$ représentant un groupe carboxy ou un groupe carboxy qui a subi une modification fonctionnelle et qui est réactif, avec un composé de formule

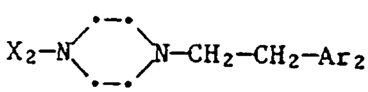

(IIb)

ou l'un de ses sels, $X_2$ représentant l'hydrogène ou un groupe protecteur du groupe amino, ou bien

b) on fait réagir un composé de formule

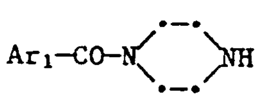

(IIIa)

ou l'un de ses sels, avec un composé de formule

$X_3-CH_2-CH_2-Ar_2$ (IIIb)

ou l'un de ses sels, $X_3$ représentant un groupe hydroxy ou un groupe hydroxy estérifié réactif, ou bien

c) on soumet un composé de formule

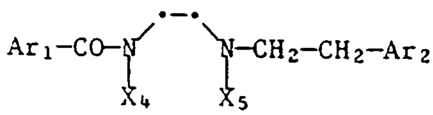

(IV)

ou l'un de ses sels, l'un des symboles $X_4$ et $X_5$ représentant l'hydrogène et l'autre un groupe de formule $-CH_2-CH_2-X_3$ dans lequel $X_3$ représente un groupe hydroxy ou un groupe hydroxy estérifié réactif, à une condensation intramoléculaire, ou bien

d) on oxyde un composé de formule

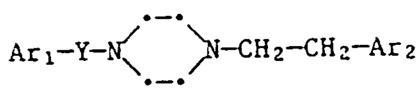

(V)

ou l'un de ses sels, Y représentant un groupe oxydable en groupe -CO-,

et si on le désire, on convertit un composé obtenu comme décrit ci-dessus ou de toute autre manière en un autre composé de formule I; on sépare un mélange d'isomères obtenu comme décrit ci-dessus en les composants, on convertit un composé de formule I à l'état libre obtenu comme décrit ci-dessus en un sel et/ou un sel obtenu comme décrit ci-dessus en le composé libre de formule I ou en un autre sel.

**2.** Procédé de préparation des composés de formule I de la revendication 1, et de leurs sels, pour lesquels $Ar_1$ représente un groupe phényle mono- ou di-substitué par des groupes alcoxy en C1-C7, caractérisé en ce que l'on alkyle un composé de formule

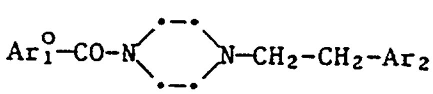

(VI)

ou l'un de ses sels, $Ar_1^o$ représentant un groupe phényle mono- ou di-substitué par des groupes hydroxy,

et si on le désire, on convertit un composé obtenu comme décrit ci-dessus ou de toute autre manière en un autre composé de formule I, on sépare un mélange d'isomères obtenu comme décrit ci-dessus en les composants, on convertit un composé de formule I obtenu à l'état libre comme décrit ci-dessus en un sel et/ou un sel obtenu comme décrit ci-dessus en le composé libre de formule I ou en un autre sel.

**3.** Procédé selon revendication 1 ou 2, caractérisé en ce que l'on prépare des composés de formule I dans laquelle $Ar_1$ représente un groupe phényle monosubstitué par un halogène de numéro atomique allant jusqu'à 35 inclus, par un groupe trifluorométhyle, par un groupe di-(alkyle en C1-C4)-amino ou par un groupe alcanoylamino en C2-C5 ou un groupe phényle disubstitué par des groupes alkyle en C1-C4 ou par des groupes alcoxy en C1-C4, et $Ar_2$ représente un groupe phényle monosubstitué par un halogène de numéro atomique allant jusqu'à 35 inclus, et leurs sels.

**4.** Procédé selon revendication 1 ou 2, caractérisé en ce que l'on prépare des composés de formule I dans laquelle $Ar_1$ représente un groupe phényle non substitué ou mono- ou di-substitué par des groupes alkyle en C1-C4, alcoxy en C1-C4, des halogènes, des groupes trifluorométhyle, di-(alkyle en C1-C4)-amino et/ou alcanoylamino en C1-C5 et $Ar_2$ un groupe phényle non substitué ou mono- ou di-substitué par des groupes alcoxy en C1-C4 ou des halogènes, et leurs sels.

5. Procédé selon revendication 1 ou 2, caractérisé en ce que l'on prépare des composés de formule I dans laquelle $Ar_1$ représente un groupe phényle non substitué ou mono- ou di-substitué bar des groupes alkyle en C1-C4, alcoxy en C1-C4, des halogènes et/ou des groupes trifluorométhyle, et $Ar_2$ représente un groupe phényle mono-substitué par un halogène, et leurs sels.

6. Procédé selon revendication 1 ou 2, caractérisé en ce que l'on prépare des composés de formule I dans laquelle $Ar_1$ représente un groupe phényle non substitué ou mono- ou di-substitué par des groupes alkyle en C1-C4, alcoxy en C1-C4, des halogènes et/ou des groupes trifluorométhyle et $Ar_2$ représente un groupe phényle mono-substitué par un groupe alcoxy en C1-C4, et leurs sels.

7. Procédé selon revendication 1 ou 2, caractérisé en ce que l'on prépare des composés de formule I dans laquelle $Ar_1$ représente un groupe 3,5-di-(alcoxy en C1-C4)-phényle et $Ar_2$ un groupe 4-halogénophényle, et leurs sels.

8. Procédé selon revendication 1 ou 2, caractérisé en ce que l'on prépare des composés de formule I dans laquelle $Ar_1$ représente un groupe 2- ou 4-fluoro-, 2- ou 4-chloro-, 2- ou 3-trifluorométhyl-, 3- ou 4-diméthylamino-, 3- ou 4-acétylamino-, 3,4- ou 3,5-diméthoxy- ou 2,6-diméthyl-phényle et $Ar_2$ un groupe 4-fluoro- ou 4-chloro-phényle, et leurs sels.

9. Procédé selon revendication 1 ou 2, caractérisé en ce que l'on prépare des composés de formule I dans laquelle $Ar_1$ représente un groupe 2- ou 4-fluoro-, 3-trifluorométhyl-, 4-chloro-, 3- ou 4-diméthylamino- ou 3- ou 4-acétylamino-phényle et $Ar_2$ un groupe 4-fluoro- ou 4-chloro-phényle, et leurs sels.

10. Procédé selon revendication 1 ou 2, caractérisé en ce que l'on prépare des composés de formule I dans laquelle $Ar_1$ représente un groupe phényle monosubstitué par un groupe méthyle, méthoxy, le fluor, le chlore ou un groupe trifluorométhyle, ou un groupe phényle disubstitué par des groupes méthoxy, et $Ar_2$ représente un groupe phényle mono-substitué par le fluor ou le chlore et leurs sels.

11. Procédé selon revendication 1 ou 2, caractérisé en ce que l'on prépare des composés de formule I dans laquelle $Ar_1$ représente un groupe phényle monosubstitué par un groupe méthyle ou le fluor et $Ar_2$ un groupe phényle mono-substitué par le fluor ou le chlore, et leurs sels.

12. Procédé selon revendication 1 ou 2, caractérisé en ce que l'on prépare des composés de formule I dans laquelle $Ar_1$ représente un groupe 2- ou 4-fluorophényle, 2-méthylphényle ou 3,5-diméthoxyphényle et $Ar_2$ un groupe 4-fluoro- ou 4-chloro-phényle, et leurs sels.

13. Procédé selon revendication 1 ou 2, caractérisé en ce que l'on prépare
la 1-[2-(4-chlorophényl)-éthyl]-4-(4-chlorobenzoyl)-pipérazine,
la 1-[2-(4-chlorophényl)-éthyl]-4-(3-chlorobenzoyl)-pipérazine,
la 1-[2-((4-chlorophényl)-éthyl]-4-(2-chlorobenzoyl)-pipérazine,
la 1-[2-(4-chlorophényl)-éthyl]-4-(2,6-dichlorobenzoyl)-pipérazine,
la 1-[2-(4-chlorophényl)-éthyl]-4-(2-fluorobenzoyl)-pipérazine,
la 1-[2-(4-chlorophényl)-éthyl]-4-(2,4-dichlorobenzoyl)-pipérazine,
la 1-[2-(4-chlorophényl)-éthyl]-4-(4-fluorobenzoyl)-pipérazine,
la 1-[2-(4-chlorophényl)-éthyl]-4-(3,4-dichlorobenzoyl)-pipérazine,
la 1-[2-(4-chlorophényl)-éthyl]-4-(3-fluorobenzoyl)-pipérazine,
la 1-[2-(4-chlorophényl)-éthyl]-4-(2-aminobenzoyl)-pipérazine,
la 1-(2-phényléthyl)-4-(2-amino-benzoyl)-pipérazine,
la 1-[2-(4-chlorophényl)-éthyl]-4-(3-méthoxy-benzoyl)-pipérazine,
la 1-[2-(4-chlorophényl)-éthyl]-4-(3,4-diméthoxybenzoyl)-pipérazine,
la 1-[2-(4-chlorophényl)-éthyl]-4-(4-méthoxybenzoyl)-pipérazine,
la 1-[2-(4-chlorophényl)-éthyl]-4-(4-méthylbenzoyl)-pipérazine,
la 1-[2-(4-chlorophényl)-éthyl]-4-(2-méthoxybenzoyl)-pipérazine,
la 1-[2-(4-chlorophényl)-éthyl]-4-(5-cyano-2-méthoxybenzoyl)-pipérazine,
la 1-[2-(4-méthoxyphényl)-éthyl]-4-(3,4-diméthoxybenzoyl)-pipérazine,
la 1-[2-(4-méthoxyphényl)-éthyl]-4-(3-méthoxybenzoyl)-pipérazine,
la 1-[2-(3-chlorophényl)-éthyl]-4-(3,4-diméthoxybenzoyl)-pipérazine,

la 1-[2-(3-chlorophényl)-éthyl]-4-(3-méthoxybenzoyl)-pipérazine,
la 1-(2-phényléthyl)-4-(3,4-diméthoxybenzoyl)-pipérazine,
la 1-(2-phényléthyl)-4-(3-méthoxybenzoyl)-pipérazine,
la 1-[2-(4-fluorophényl)-éthyl]-4-(3,4-diméthoxybenzoyl)-pipérazine,
la 1-[2-(4-fluorophényl)-éthyl]-4-(4-chlorobenzoyl)-pipérazine,
la 1-[2-(4-fluorophényl)-éthyl]-4-(4-fluorobenzoyl)-pipérazine,
la 1-[2-(4-fluorophényl)-éthyl-4-(3-méthoxybenzoyl)-pipérazine,
la 1-[2-(4-chlorophényl)-éthyl]-4-(2-trifluorométhylbenzoyl)-pipérazine,
la 1-[2-(4-chlorophényl)-éthyl]-4-(3-trifluorométhylbenzoyl)-pipérazine,
la 1-[2-(4-chlorophényl)-éthyl]-4-(4-trifluorométhylbenzoyl)-pipérazine,
la 1-[2-(4-chlorophényl)-éthyl]-4-(4-méthylbenzoyl)-pipérazine,
la 1-[2-(4-chlorophényl)-éthyl]-4-(2-méthylbenzoyl)-pipérazine,
la 1-[2-(4-chlorophényl)-éthyl]-4-(2,6-diméthylbenzoyl)-pipérazine,
la 1-[2-(4-chlorophényl)-éthyl]-4-(3,5-dichlorobenzoyl)-pipérazine,
la 1-[2-(4-chlorophényl)-éthyl]-4-(2,6-diméthoxybenzoyl)-pipérazine,
ou un de leurs sels.

**14.** Procédé selon revendication 1, caractérisé en ce que l'on prépare la 1-[2-(4-chlorophényl)-éthyl]-4-(2-fluorobenzoyl)pipérazine ou l'un de ses sels.

**15.** Procédé selon revendication 1, caractérisé en ce que l'on prépare la 1-[2-(4-chlorophényl)-éthyl]-4-(3-diméthylaminobenzoyl)pipérazine ou l'un de ses sels.

**16.** Procédé selon revendication 1, caractérisé en ce que l'on prépare la 1-[2-(4-chlorophényl)-éthyl]-4-(4-diméthylaminobenzoyl)pipérazine ou l'un de ses sels.

**17.** Procédé selon revendication 1, caractérisé en ce que l'on prépare la 1-[2-(4-chlorophényl)-éthyl]-4-(4-acétaminobenzoyl)pipérazine ou l'un de ses sels.

**18.** Procédé selon revendication 1, caractérisé en ce que l'on prépare la 1-[2-(4-chlorophényl)-éthyl]-4-(3,5-diméthoxybenzoyl)pipérazine ou l'un de ses sels.

**19.** Procédé selon revendication 1, caractérisé en ce que l'on prépare la 1-[2-(p-chlorophényl)-éthyl]-4-(3-acétamidobenzoyl)pipérazine ou l'un de ses sels.

**20.** Procédé de préparation de compositions pharmaceutiques, caractérisé en ce que l'on met un composé selon l'une des revendications 1 à 19, à l'état libre ou à l'état de sel acceptable pour l'usage pharmaceutique, sous forme d'une composition pharmaceutique, éventuellement en mélangeant avec des produits auxiliaires et véhicules usuels.

**21.** Composés de formule

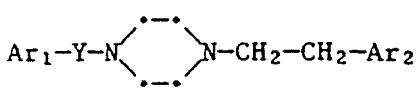

(V)

dans laquelle Y représente $-CH_2-$ et $Ar_1$ et $Ar_2$ ont les significations indiquées dans l'une des revendications 1 à 12, et leurs sels.